(19) Europäisches Patentamt
European Patent Office
Office européen des brevets

(11) **EP 4 582 045 A1**

(12) **EUROPEAN PATENT APPLICATION**
published in accordance with Art. 153(4) EPC

(43) Date of publication:
**09.07.2025 Bulletin 2025/28**

(21) Application number: **22957421.5**

(22) Date of filing: **31.08.2022**

(51) International Patent Classification (IPC):
***A61B 34/37*** *(2016.01)*

(52) Cooperative Patent Classification (CPC):
**A61B 34/37**

(86) International application number:
**PCT/JP2022/032883**

(87) International publication number:
**WO 2024/047824 (07.03.2024 Gazette 2024/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Asahi Surgical Robotics Co.,Ltd.
Kashiwa-shi, Chiba 277-8577 (JP)**

(72) Inventors:
• **AWANO, Keita
Kashiwa-shi, Chiba 277-8577 (JP)**
• **ANDO, Takehiro
Kashiwa-shi, Chiba 277-8577 (JP)**
• **MIYAMOTO, Hiroyuki
Kashiwa-shi, Chiba 277-8577 (JP)**

(74) Representative: **Winter, Brandl - Partnerschaft
mbB
Alois-Steinecker-Straße 22
85354 Freising (DE)**

(54) **MANIPULATOR**

(57) A manipulator attachable to and detachable from a medical device includes: a manipulator main body having a distal end portion attached with an end effector and a proximal end portion connectable to the medical device; and a mechanical counter mechanism disposed on the manipulator main body. The counter mechanism shifts from a first state to a second state by a first operation for the counter mechanism and shifts from the second state to the first state by a second operation for the counter mechanism, and counts the shifts from the second state to the first state by the second operation.

Fig. 1

## Description

TECHNICAL FIELD

The disclosed embodiments relate to a manipulator.

BACKGROUND ART

Laparoscopic surgeries have been known, in which a rod-shaped surgical instrument is inserted into a body cavity of a patient through a hole opened on an abdomen of the patient for surgery. This surgical instrument is also referred to as a manipulator, which has, on its distal end portion, a movable portion referred to as an end effector capable of rotating and grasping a biological tissue. For example, Patent Literatures 1 to 3 describe the above-described manipulator. For example, Patent Literature 4 describes a rack used for washing the above-described manipulator.

CITATION LIST

Patent Literature

   Patent Literature 1: US 2002/0032452 A
   Patent Literature 2: JP 2011-194129 A
   Patent Literature 3: Japanese Patent Publication No. 5830258
   Patent Literature 4: Japanese Patent Publication No. 6472532

SUMMARY OF INVENTION

TECHNICAL PROBLEM

Since a manipulator is inserted into a body cavity of a patient for use, the inside and outside of the manipulator needs to be washed and sterilized for every use. In this case, since the manipulator deteriorates in association with the use, the manipulator may cause failures resulting from the deterioration due to repeated uses. In particular, the manipulator has great concerns for failures because the movable portion of the end effector has a complicated mechanism and the manipulator is exposed to a severe environment in a sterilization process. In order to suppress such failures, generally, an upper limit count of the uses of the manipulator is determined, the uses are counted for every reuse. When the count of the uses reaches the upper limit, the manipulator is discarded. However, if the uses of the manipulator are not accurately counted (in particular, if the uses are not counted even though the manipulator has been used), the manipulator is reused even though the usage count exceeds the upper limit, and therefore there has been a concern that failures occur during the procedure.

In this regard, in the technique described in Patent Literature 1, the manipulator is physically and electrically connected to a medical device, and the remaining uses of the manipulator are counted by providing a scale on the manipulator side. However, the technique described in Patent Literature 1 has had a problem that the remaining uses cannot be confirmed unless the manipulator is connected to the medical device, and a problem that the scale or an electrical connection portion may cause failures due to a severe sterilization process. In the technique described in Patent Literature 2, the remaining uses are counted by converting a thermal energy received in a sterilization process of the manipulator into a mechanical energy using a shape memory member. However, the technique described in Patent Literature 2 has had problems that, due to the environmental factors such as the outside air temperature, which affect the thermal conditions of the sterilization process, there is no guarantee that the shape of the shape memory member reliably changes during the sterilization process, and the counting cannot be reliably performed. Furthermore, in the technique described in Patent Literature 3, when a manipulator is connected to a medical device, the remaining uses are counted by driving a dial incorporated into the manipulator. However, the technique described in Patent Literature 3 has had a problem that the uses are counted even when the manipulator and the medical device are attached to or detached from each other unintentionally by the user (e.g. when the connection between the manipulator and the medical device is insufficient and the connecting operation is tried several times), resulting in low counting accuracy. In Patent Literature 4, these problems are not taken into account.

The disclosed embodiments have been made to solve at least a part of the above problems, and an object of the disclosed embodiments is to provide a technique of accurately counting the uses of the manipulator.

SOLUTION TO PROBLEM

The disclosed embodiments have been made to solve at least a part of the above problems, and can be embodied as the following aspects.

   (1) According to an aspect of the disclosed embodiments, a manipulator attachable to and detachable from a medical device is provided. This manipulator includes: a manipulator main body having a distal end portion attached with an end effector and a proximal end portion connectable to the medical device; and a mechanical counter mechanism disposed on the manipulator main body, in which the counter mechanism shifts from a first state to a second state by a first operation for the counter mechanism and shifts from the second state to the first state by a second operation for the counter mechanism, and counts the shifts from the second state to the first state by the second operation.

According to this configuration, the counter mechanism shifts from the first state to the second state by the first operation for the counter mechanism, and shifts from the second state to the first state by the second operation for the counter mechanism after the shift to the second state. Then, the counter mechanism counts the shifts from the second state to the first state by the second operation. That means, the counter mechanism has the first state of impossible counting and the second state of possible counting, and counts only the shifts from the second state to the first state, to makes it possible to prevent miscounting. By using such a counter mechanism for counting the uses of the manipulator, the uses of the manipulator can be counted with high accuracy.

(2) The manipulator according to the above aspect may be configured such that the counter mechanism includes: a counter disk that indicates a count number on its main surface and is rotatably fixed to the manipulator main body; a first member that is rotatably fixed to the manipulator main body and rotates to one side in a rotational direction to rotate the counter disk, and rotates to the other side not to rotate the counter disk; and a second member that engages with the first member to restrict the first member to rotate to the other side, in which, in the first state, the second member and the first member engage with each other and the first member rotates relatively to the one side, and in the second state, the second member and the first member do not engage with each other and the first member rotates relatively to the other side.

According to this configuration, a mechanical structure composed of the counter disk, the first member, and the second member rotates the first member of the counter mechanism relatively to the one side during the shift from the second state to the first state to rotate the counter disk, so that the shifts can be automatically counted.

(3) The manipulator according to the above aspects may be configured such that, in the first state of the counter mechanism, an end portion of the first member is accommodated in the manipulator main body to allow the proximal end portion of the manipulator to be connected with the medical device, and in the second state of the counter mechanism, the end portion of the first member protrudes outside the manipulator main body to prevent the proximal end portion of the manipulator from being connected to the medical device.

According to this configuration, since the end portion of the first member is accommodated in the manipulator main body in the first state and the end portion of the first member protrudes outside the manipulator main body in the second state, the user can grasp the current state of the manipulator (whether the first state or the second state, i.e. whether before or after the counter) at first sight. According to this configuration, since the end portion of the first member protrudes outside the manipulator main body to prevent the proximal end portion of the manipulator from being connected to the medical device in the second state, the transitions i.e. the shifts from the second state to the first state can be prevented from being missed to be counted.

(4) The manipulator according to the above aspects may be configured such that, when the shifts from the second state to the first state reach a predetermined count, the counter mechanism is prevented from transitioning from the second state to the first state.

According to this configuration, the counter mechanism is prevented from transitioning from the second state to the first state when the shifts from the second state to the first state reach a predetermined count. In the second state, since the end portion of the first member protrudes outside the manipulator main body to prevent the proximal end portion of the manipulator from being connected to the medical device, the user can be prevented from erroneously attaching the manipulator that has been used over an upper limit count of the shifts from the second state to the first state (i.e. the count of the uses of the manipulator) to the medical device and reusing it.

(5) The manipulator according to the above aspects may be configured such that the counter mechanism further has an indicating portion that indicates the count of the shifts.

According to this configuration, since the counter mechanism has the indicating portion that indicates the count of the shifts, the user can easily confirm the count of the shifts from the second state to the first state (i.e. the count of the uses of the manipulator 2) by checking the indicating portion. As a result, usability of the manipulator can be improved.

The disclosed embodiments can be made in various aspects, e.g. in forms of a manipulator attachable to and detachable from a medical device such as a surgery supporting robot, a manipulator assisting device connected to the manipulator, a washing nozzle for washing the manipulator, a manipulator assisting device including the washing nozzle, a manipulator system including the manipulator assisting device and the manipulator, a surgery supporting robot including the manipulator assisting device or the manipulator, and a manufacturing method therefor.

BRIEF DESCRIPTION OF DRAWINGS

Fig. 1 is an explanatory diagram illustrating a configuration of a manipulator system.
Fig. 2 is an explanatory diagram illustrating a configuration of an end effector.
Fig. 3 is an explanatory diagram illustrating a configuration of a main body device viewed from a proximal end side.
Fig. 4 is an explanatory diagram illustrating a state transition of an assisting device.

Fig. 5 is an explanatory diagram illustrating a configuration of the assisting device viewed from the proximal end side.

Fig. 6 is an explanatory diagram illustrating a configuration of the assisting device viewed from a distal end side.

Fig. 7 is an exploded perspective view of the assisting device.

Fig. 8 is an explanatory diagram illustrating connection of the assisting device with a manipulator.

Figs. 9A to 9D are diagrams illustrating states of the assisting device in each mode illustrated in Fig. 4.

Figs. 10A and 10B are diagrams illustrating a relationship between a dial and a main shaft in each mode illustrated in Fig. 4.

Fig. 11 is a diagram illustrating a relationship between an assisting gear and a driven gear in each mode illustrated in Fig. 4.

Figs. 12A and 12B are explanatory longitudinal sectional views of the assisting device.

Figs. 13A and 13B are explanatory diagrams illustrating a configuration of a counter mechanism in the manipulator.

Fig. 14 is an explanatory diagram illustrating an actuator that causes the counter mechanism to operate.

Fig. 15 is an explanatory diagram illustrating a configuration of a washing nozzle.

Figs. 16A to 16C are explanatory diagrams illustrating a configuration of a washing nozzle.

Figs. 17A and 17B are explanatory diagrams illustrating a method for using the washing nozzle.

Fig. 18 is an explanatory diagram illustrating a configuration of a manipulator according to the second embodiment.

Figs. 19A to 19C are diagrams illustrating a state of an assisting device in each mode according to the second embodiment.

Fig. 20 is a diagram illustrating a relationship between an assisting gear and a driven gear in each mode according to the third embodiment.

Fig. 21 is an explanatory diagram illustrating an assisting device and a manipulator according to the fourth embodiment.

Fig. 22 is an explanatory diagram illustrating a state transition of an assisting device according to the fifth embodiment.

Fig. 23 is an explanatory diagram illustrating a configuration of a washing nozzle according to the sixth embodiment.

Fig. 24 is an explanatory diagram illustrating a configuration of a washing nozzle according to the seventh embodiment.

EMBODIMENTS OF THE INVENTION

<First Embodiment>

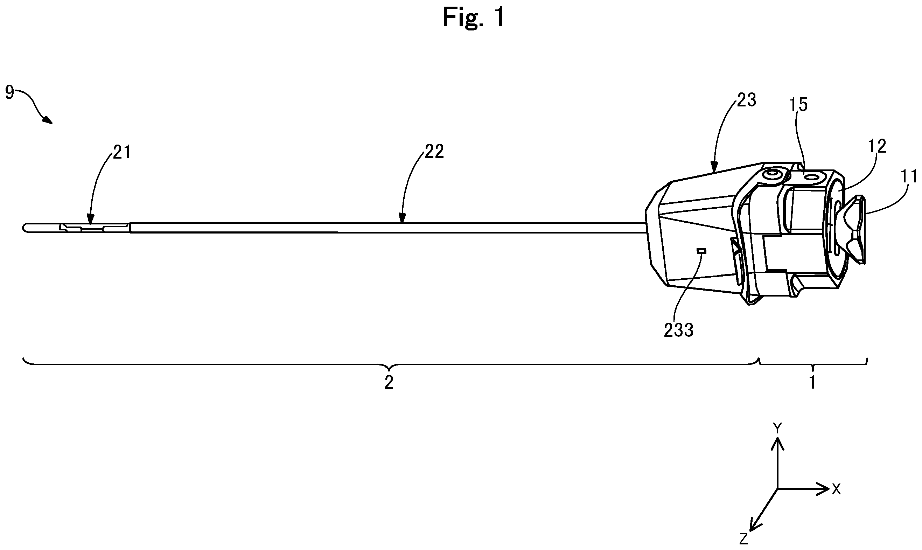

Fig. 1 is an explanatory diagram illustrating a configuration of a manipulator system 9. The manipulator system 9 includes a manipulator 2 and a manipulator assisting device 1 (hereinafter, also simply referred to as an "assisting device 1"). The manipulator 2 is attached to a robot arm of a surgery supporting robot (medical device) and used in laparoscopic surgery or the like. The assisting device 1 assists use of the manipulator 2. The manipulator 2 may be configured so as to be attached to a medical device that is directly operated by a user instead of a surgery supporting robot (medical device). The assisting device 1 has functions roughly sorted into the following functions a, b, and c. The detailed functions will be described later.

(a) Function of assisting removal of the manipulator 2 from a body cavity of a patient.
(b) Function of counting uses of the manipulator 2.
(c) Function of assisting washing of the inside of the manipulator 2.

For convenience of explanation, Fig. 1 includes portions illustrated such that a relative ratio of sizes between the constituents is different from the actual ratio. Fig. 1 includes portions where a part of each constituent member is exaggeratedly illustrated. In Fig. 1, mutually-orthogonal X, Y, and Z axes are illustrated. The X axis corresponds to the longitudinal direction of the assisting device 1 and the manipulator 2, the Y axis corresponds to the width direction of the assisting device 1 and the manipulator 2, and the Z axis corresponds to the height direction of the assisting device 1 and the manipulator 2. In Fig. 1, the left side (-X axis direction) is referred to as a "distal end side" of the assisting device 1 and the manipulator 2, and the right side (+X axis direction) is referred to as a "proximal end side" of the assisting device 1 and the manipulator 2. Of both ends in the longitudinal direction (X axis direction) of the assisting device 1 and the manipulator 2, one end located on the distal end side is referred to as a "distal end", and the other end located on the proximal end side is referred to as a "proximal end". The distal end and its vicinity are referred to as a "distal end portion", and the proximal end and its vicinity are referred to as a "proximal end portion". These definitions are common to the figures following Fig. 1.

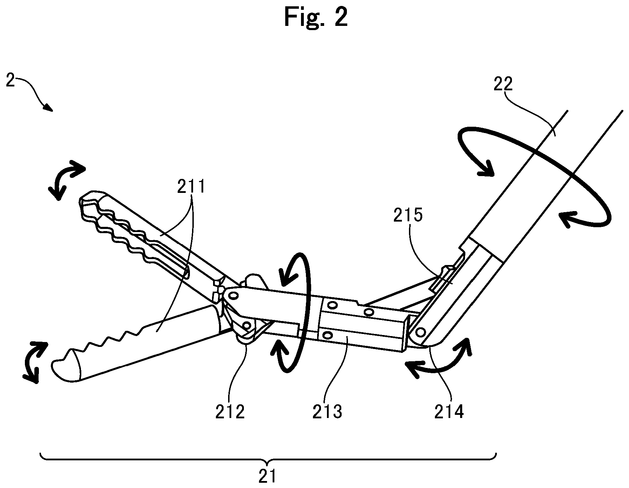

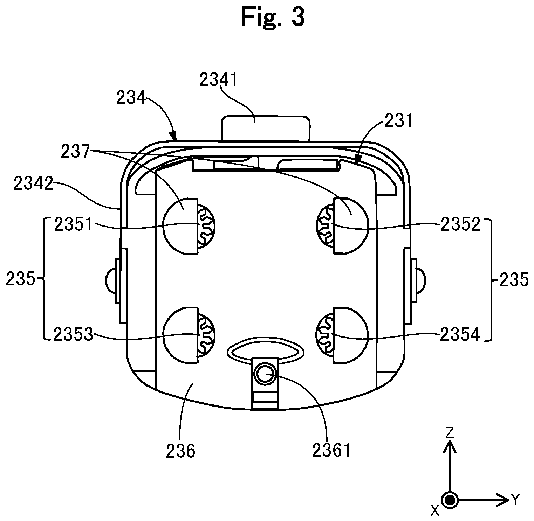

Fig. 2 is an explanatory diagram illustrating a configuration of an end effector 21. Fig. 3 is an explanatory diagram illustrating a configuration of a main body device 23 viewed from a proximal end side. As illustrated in Fig. 1, the manipulator 2 has the end effector 21, a main shaft 22, and a main body device 23 in this order from the distal end side toward the proximal end side. In other words, the end effector 21 is attached to the distal end portion of the main body device 23 (specifically, a manipulator main body 231) via the main shaft 22.

The end effector 21 is a movable portion disposed on the distal end portion of the manipulator 2. As illustrated in Fig. 2, the end effector 21 includes forceps 211, a first joint 212, a first extending portion 213, a second joint 214, and a second extending portion 215. The forceps 211 includes two long narrow grasping members that grasp a biological tissue by separating/-contacting their distal ends from/with each other while the proximal ends of these grasping members are fixed. In the example of Fig. 2, the contact surfaces on the distal end portions of the grasping members are formed in a wave shape for facilitating grasping of a biological tissue. The first joint 212 is disposed between the forceps 211 and the first extending portion 213. The first joint 212 fixes the forceps 211 to the first extending portion 213 in such a state that the forceps 211 are rotatable. The first extending portion 213 is disposed between the first joint 212 and the second joint 214. The second joint 214 is disposed between the first extending portion 213 and the second extending portion 215. The second joint 214 fixes the first extending portion 213 to the second extending portion 215 in such a state that the first extending portion 213 are bendable. The second extending portion 215 is disposed on the proximal end side relative to the second joint 214.

The main shaft 22 is a rod-shaped member disposed between the end effector 21 and the main body device 23. As illustrated in Fig. 2, the main shaft 22 is axially rotatable. A power transmission member for operating each portion of the end effector 21 described above is accommodated inside the main shaft 22.

The main body device 23 is disposed on the proximal end portion of the manipulator 2. The main body device 23 is attachable to and detachable from a surgery supporting robot or the assisting device 1. As illustrated in Fig. 3, the main body device 23 includes a manipulator main body 231 as a chassis constituting the main body of the main body device 23. On the proximal end surface 236 of the manipulator main body 231, four driven gears 235 that transmit power to four power transmission members of the main body device 23 respectively, and a washing port 2361. The washing port 2361 is an opening that communicates with a flow passage formed inside the main body device 23 and the main shaft 22. The inside of the manipulator 2 is sealed except for the washing port 2361 and the forceps 211 of the end effector 21. In other words, a fluid cannot enter and exit the inside of the manipulator 2 excluding the washing port 2361 and the forceps 211. From the viewpoint of improving sealability for the inside of the manipulator 2, the smaller the number of openings provided in the manipulator 2, the better. For this reason, the manipulator 2 according to the first embodiment has only one opening (the washing port 2361) except for the forceps 211. The manipulator 2 may have a removable cover for closing the washing port 2361.

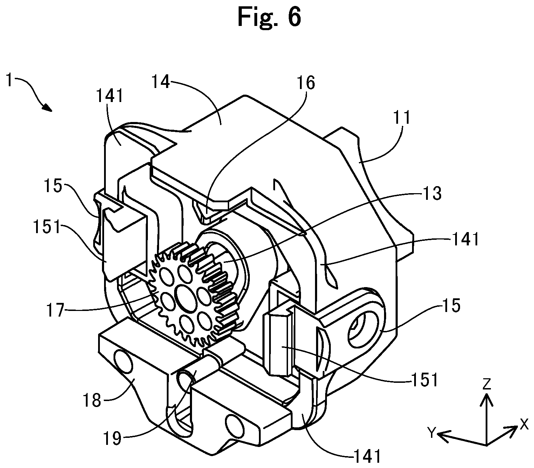

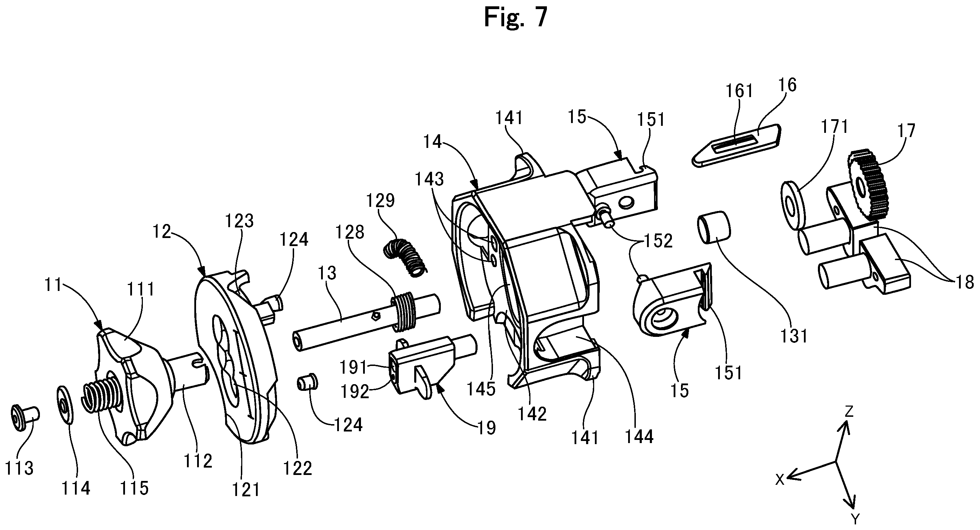

The driven gears 235 include a grasping shaft gear 2351, a main shaft gear 2352, a distal end rotation shaft gear 2353, and a bending shaft gear 2354. The grasping shaft gear 2351 transmits power for switching between the release state and the grasp state of the forceps 211. The main shaft gear 2352 transmits power for rotating the main shaft 22. The distal end rotation shaft gear 2353 transmits power for rotating the forceps 211. The bending shaft gear 2354 transmits power for bending the second joint 214. Each of these driven gears 235 is partially (about half in the example of Fig. 3) covered by a gear cover 237, and the remaining portion is exposed to the outside. The driven gears 235 engage with a gear of the surgery supporting robot when the manipulator 2 is connected to the surgery supporting robot. The driven gears 235 engage with an assisting gear 17 (Fig. 6 and Fig. 7) of the assisting device 1 when the manipulator 2 is connected to the assisting device 1. Other components of the manipulator 2 will be described later. The grasping shaft gear 2351 functionally serves as a "first driven gear", and the bending shaft gear 2354 functionally serves as a "second driven gear".

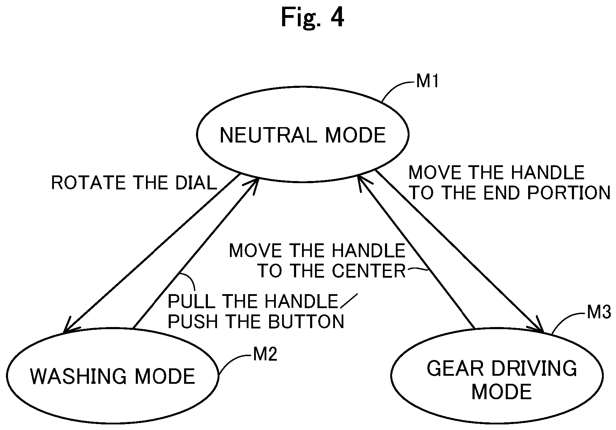

Fig. 4 is an explanatory diagram illustrating a state transition of the assisting device 1. The assisting device 1 has three operation modes illustrated in Fig. 4. The washing mode M2 executes the (c) function of assisting washing of the inside of the manipulator 2. The gear driving mode M3 executes the (a) function of assisting removal of the manipulator 2. The neutral mode M1 is a mode that necessarily intermediate the switching between the washing mode M2 and the gear driving mode M3, and the neutral mode has no executable function. Transition ways between the modes will be described later.

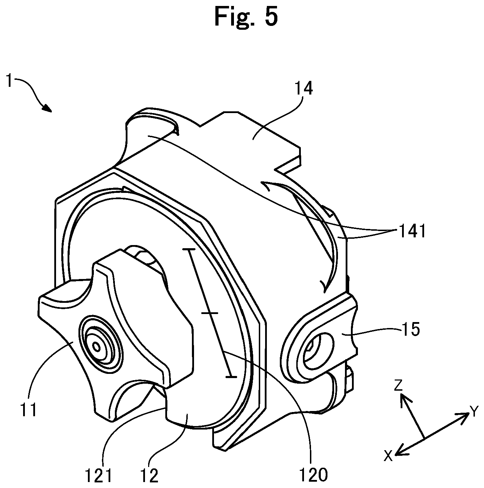

Fig. 5 is an explanatory diagram illustrating a configuration of the assisting device 1 viewed from the proximal end side. Fig. 6 is an explanatory diagram illustrating a configuration of the assisting device 1 viewed from the distal end side. Fig. 7 is an exploded perspective view of the assisting device 1. The configuration of the assisting device 1 will be explained with reference to Fig. 5 to Fig. 7. The assisting device 1 includes a handle 11, a dial 12, a main shaft 13, a frame 14, side lock buttons 15, a pin 16, the assisting gear 17, a protruding portion 18, and a washing nozzle 19. The assisting device 1 corresponds to the "manipulator assisting device main body".

The handle 11 is disposed on the most proximal end side of the assisting device 1 and used for a user to operate the assisting device 1. The handle 11 is connected to the assisting gear 17 via the main shaft 13 passing through the frame 14 to transmit a drive force to the assisting gear 17. The handle 11 includes a grip portion 111 to be gripped by a user, and a cylindrical extending portion 112 that extends from the grip portion 111 toward the distal end side (i.e. the dial 12 side). The handle 11 is fixed by screwing a screw 113 into the inside of the main shaft 13 via a washer 114 while the main shaft 13 is inserted into the extending portion 112 and the knob latch spring 115 is mounted in the grip portion 111. As a result, the handle 11 is fixed while energized toward the distal end side (i.e. the dial 12 side).

The dial 12 functionally serves as a "switching portion" that switches between the neutral mode M1, the washing mode M2, and the gear driving mode M3, explained in Fig. 4. The dial 12 has a disk shape, i.e. a circular plate shape. The dial 12 is disposed between the handle 11 and the frame 14. The dial 12 has a notch 121 with a part of the outer periphery recessed toward the center of the dial 12, and a second elongated hole 122 provided in the center of the dial 12. The second elongated hole 122 is an elliptical through hole that penetrates the distal end surface and the proximal end surface of the dial 12. The dial 12 is fixed to the frame 14 while the main shaft 13 is inserted into the second elongated hole 122. Thus, the dial 12 is rotatable around the main shaft 13. A cam structure portion 123 for operating the pin 16 is disposed on a peripheral edge portion of the dial 12 on the distal end side (the frame 14 side). The cam structure portion 123 will be described later in detail. Two dial lock pins 124 are disposed on the distal end-side surface (the frame 14 side) of the dial 12. The two dial lock pins 124 are protruding portions that protrude toward the distal end side (the frame 14 side). A position indicator 120 is provided on the proximal end-side surface (the handle 11 side) of the dial 12. The position indicator 120 is provided in a scale notation, in which one end of the scale is located at a position corresponding to one end of the second elongated hole 122, the other end of the scale is located at a position corresponding to the other end of the second elongated hole 122, and the center of the scale is located at a position corresponding to the center of the second elongated hole 122.

The main shaft 13 is a hollow cylindrical member extending in the longitudinal direction (X axis direction) of the assisting device 1. The main shaft 13 passes through the handle 11, the dial 12, and the frame 14 in this order from the proximal end side toward the distal end side. An assisting gear 17 is fixed to the distal end portion of the main shaft 13 via a bearing 131 and a washer 171. A spring 128 extending in the X axis direction and a dial rotation spring 129 extending in the YZ axis direction are disposed between the frame 14 and the dial 12. The spring 128 is arranged so as to surround the periphery of the main shaft 13. The dial 12 is energized toward the proximal end side (i.e. the handle 11 side) by the spring 128. The dial 12 is energized in the circumferential direction (YZ axis direction) of the dial 12 by the dial rotation spring 129. The main shaft 13 may have a circular columnar shape, a polygonal columnar shape, or a polygonal cylindrical shape as long as it is rod-shaped.

The frame 14 is a chassis constituting the main body of the assisting device 1 and functionally serves as a "main body portion". The frame 14 is disposed between the dial 12 and the assisting gear 17. A first elongated hole 145 is formed at the center of the frame 14. The first elongated hole 145 is an elliptical through hole that penetrates the distal end surface and the proximal end surface of the frame 14. The main shaft 13 passes through the first elongated hole 145. The frame 14 has a bottomed cylindrical shape with a bottom and a raised edge on the proximal end side (the dial 12 side). The bottom of the frame 14 is also referred to as a "proximal end surface 142". The proximal end surface 142 has two pairs of two recessed portions 143. In the assembled state of the assisting device 1, the dial 12 is accommodated inside the edge (Fig. 5). In the assembled state, the dial lock pin 124 of the dial 12 is fitted into one of the recessed portions 143, so that the dial 12 is fixed to the frame 14 in a state that the dial 12 relatively rotates. The dial lock pin 124 of the dial 12 is fitted into the other one of the recessed portions 143, so that the dial 12 is fixed to the frame 14 in a state that the dial 12 does not relatively rotate.

On the distal end side (the assisting gear 17 side) of the frame 14, flanges 141 whose four corners are raised outward in the circumferential direction are formed (i.e. a total of four flanges 141 are formed at the four corners). Two accommodation portions 144 are formed between one flange 141 and the other flange 141. The two accommodation portions 144 are recessed portions formed on opposing positions of the frame 14 ($\pm$Y axis direction of the frame 14). Each side lock button 15 is assembled to each of the accommodation portions 144 (Fig. 5 and Fig. 6).

The side lock button 15 refers to a member for maintaining and releasing the connection between the assisting device 1 and the manipulator 2. The side lock button 15 has a shaft 152 raised toward the frame 14 side. The side lock button 15 is fixed to the frame 14 by fitting the shaft 152 into a shaft hole (not illustrated) provided in the accommodation portion 144 of the frame 14. A claw 151 protruding outward is disposed on the distal end of the side lock button 15 i.e. on the outside of the side lock button 15 (the side opposite to the frame 14).

The pin 16 is a long narrow member having a flat plate shape, which functionally serves as an "actuator" that switches the counter mechanism (described later in detail) of the manipulator 2 to a decrementable state. An elongated hole 161 extending along the extending direction of the pin 16 and penetrating one surface and the other surface of the pin 16 is formed at the center of the pin 16. A spring not illustrated is disposed in the elongated hole 161 of the pin 16. The spring is arranged such that its one end is in contact with the frame 14 and the other end presses the proximal end side (the handle 11 side) of a portion demarcating the elongated hole 161 of the pin 16. The pin 16 is energized toward the proximal end side (the handle 11 side) by the spring.

The assisting gear 17 engages with the driven gears 235 of the manipulator 2 when the assisting device 1 is connected to the manipulator 2. The assisting gear 17 is disposed on the distal end side of the frame 14. The assisting gear 17 is fixed to the distal end portion of the main shaft 17 via the bearing 131 and the washer 171. Thus, the assisting gear 17 rotates in the same direction as the rotational direction of the handle 11 when the user rotates the handle 11 to transmit the rotational force to the assisting gear 17 via the main shaft 13.

The protruding portion 18 is attached to the distal end-side surface of the frame 14 to protrude a part of the frame 14 toward the distal end side. By the protruding portion 18, a space for allowing the assisting gear 17 to rotate can be formed between the frame 14 of the assisting device 1 and the manipulator 2 when the assisting device 1 is connected to the manipulator 2 (Fig. 6).

The washing nozzle 19 is a member that is connected to the flow passage in the manipulator 2 when the assisting device 1 is connected to the manipulator 2, to feed a washing fluid (e.g. a washing liquid for washing a medical device) to the flow passage in the manipulator 2. In other words, the washing nozzle 19 is a member for washing the manipulator 2. On the proximal end-side surface of the washing nozzle 19, a first proximal end opening 191 and a second proximal end opening 192, to which an external instrument (syringe) is connected, are provided. The washing nozzle 19 will be described later in detail.

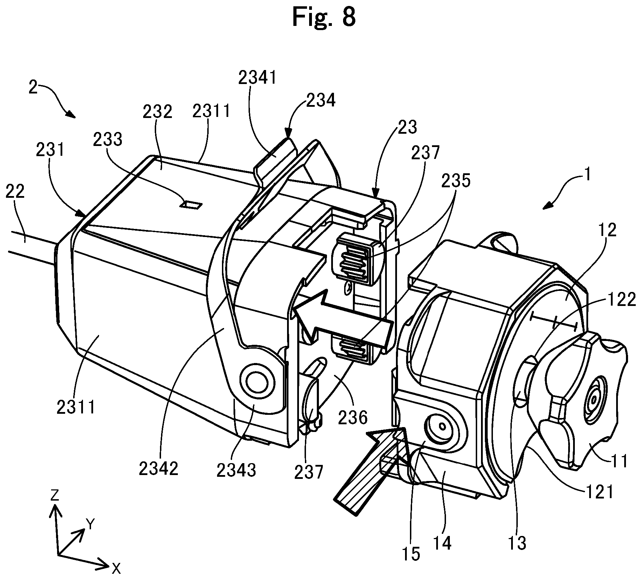

Fig. 8 is an explanatory diagram illustrating the connection between the assisting device 1 and the manipulator 2. As illustrated in Fig. 8, in the main body device 23 of the manipulator 2, a lid 232 is provided on one surface of the manipulator main body 231. A counter mechanism (described later in detail) is accommodated inside the lid 232. A window 233 for indicating the "count" counted by the counter mechanism is disposed at the center of the lid 232. The window 233 is a through hole penetrating the outer surface and the inner surface of the lid 232. A lever 234 is further attached to the main body device 23. The lever 234 has a projecting portion 2341 to be gripped by a user, and a pair of arms 2342 individually extending toward the side surfaces 2311 of the manipulator main body 231. In the lever 234, end portions 2343 of the arms 2342 are rotatably fixed to the side surfaces 2311 of the manipulator main body 231. The side surfaces 2311 are adjacent to the surface of the manipulator main body 231 where the lid 232 is provided. As described above, the four driven gears 235 are disposed on the proximal end surface 236 of the manipulator main body 231.

As illustrated in Fig. 8, the distal end portion of the assisting device 1 is attachable to and detachable from the proximal end portion of the manipulator 2. Specifically, as indicated by a white blank arrow, when the assisting device 1 is pushed to the manipulator 2, the claw 151 of the side lock button 15 is caught by the manipulator 2, so that the assisting device 1 can maintain the connection between the assisting device 1 and the manipulator 2. As indicated by a hatched arrow, when the user pushes the side lock button 15 toward the frame 14 side, the engagement between the claw 151 and the manipulator 2 can be released to release the connection between the assisting device 1 and the manipulator 2.

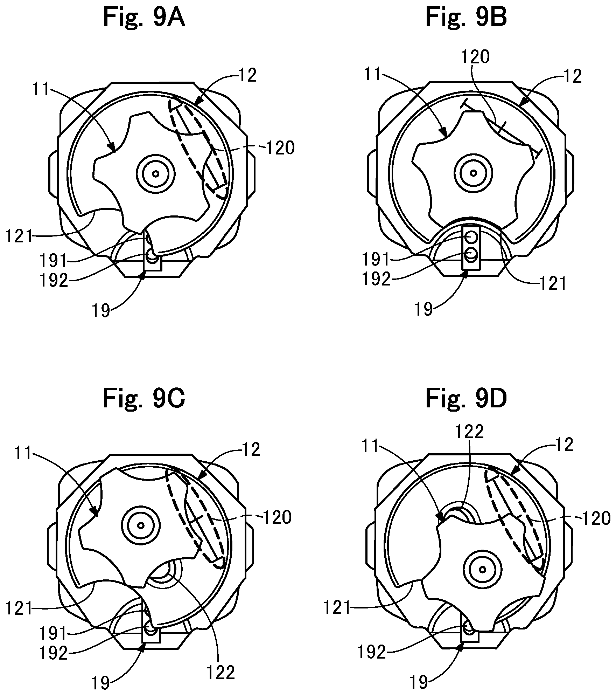

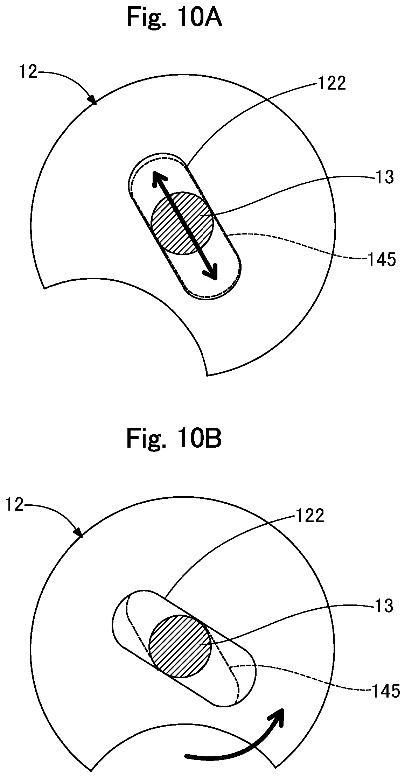

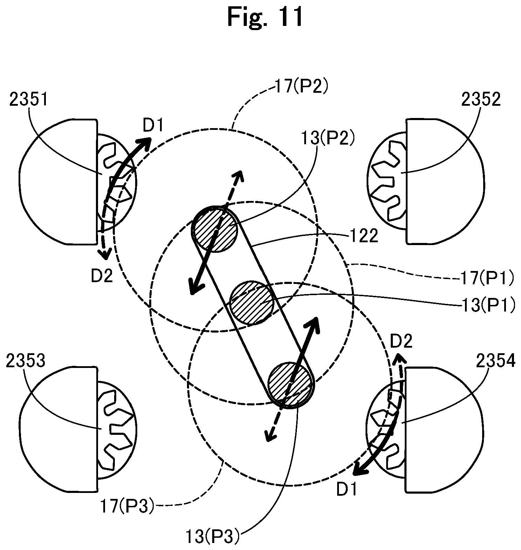

Figs. 9A to 9D are diagrams illustrating states of the assisting device 1 in each mode illustrated in Fig. 4. Figs. 10A and 10B are diagrams illustrating a relationship between the dial 12 and the main shaft 13 in each mode illustrated in Fig. 4. Fig. 11 is a diagram illustrating a relationship between the assisting gear 17 and the driven gears 235 in each mode illustrated in Fig. 4. The driving mode of the assisting device 1 will be explained with reference to Fig. 4, and Fig. 9A to Fig. 11.

Fig. 9A is a diagram illustrating a state of the assisting device 1 in the neutral mode M1. Fig. 10A is a diagram illustrating the dial 12 at the first position. In the neutral mode M1, the openings (the first proximal end opening 191 and the second proximal end opening 192) of the washing nozzle 19 are closed by the dial 12. Thereby, the washing nozzle 19 is restricted to be accessed. In the neutral mode M1, as illustrated in Fig. 11, the handle 11 and the main shaft 13 connected to the handle 11 are located at the center P1 of the second elongated hole 122 of the dial 12. Thus, the assisting gear 17 (P1) located on the distal end portion of the main shaft 13 is not engaged with any of the driven gears 235 of the manipulator 2. Furthermore, in the neutral mode M1, as illustrated in Fig. 10A, the second elongated hole 122 of the dial 12 is oriented in the same direction as of the first elongated hole 145 of the frame 14 (the extending direction of the longitudinal axis of the second elongated hole 122 coincides with the extending direction of the longitudinal axis of the first elongated hole 145). Thus, the handle 11 and the main shaft 13 connected to the handle 11 are allowed to slide. The handle 11 and the main shaft 13 slide on the YZ plane intersecting the main shaft 13 (X axis).

Fig. 9B is a diagram illustrating a state of the assisting device 1 in the washing mode M2. Fig. 10B is a diagram illustrating the dial 12 at the second position. In the washing mode M2, the position of the notch 121 of the dial 12 coincides with the positions of the openings (the first proximal end opening 191 and the second proximal end opening 192) of the washing nozzle 19. Thereby, the washing nozzle 19 is allowed to be accessed. Furthermore, in the washing mode M2, as illustrated in Fig. 10B, the second elongated hole 122 of the dial 12 is oriented in the different direction from that of the first elongated hole 145 of the frame 14 (the extending direction of the longitudinal axis of the second elongated hole 122 differs from the extending direction of the longitudinal axis of the first elongated hole 145). Thus, the restriction of the movement of the main shaft 13 by the dial 12 and the frame 14 restricts the handle 11 and the main shaft 13 connected to the handle 11 to slide. In other words, in Fig. 10B, the phases of the second elongated hole 122 and the first elongated hole 145 are deviated from each other, so that the handle 11 and the main shaft 13 are restricted to slide.

Fig. 9C and Fig. 9D are diagrams illustrating a state of the assisting device 1 in the gear driving mode M3. In the gear driving mode M3, the openings (the first proximal end opening 191 and the second proximal end opening 192) of the washing nozzle 19 are closed by the dial 12. Thereby, the washing nozzle 19 is restricted to be accessed. Furthermore, in the gear driving mode M3, as illustrated in Fig. 10A, the second elongated hole 122 of the dial 12 is oriented in the same direction as of the first elongated hole 145 of the frame 14. Thus, the handle 11 and the main shaft 13 connected to the handle 11 are allowed to slide.

As illustrated in Fig. 9C and Fig. 11, in the neutral mode M1, when the handle 11 slides to one end side of the second elongated hole 122, the main shaft 13 connected to the handle 11 slides to one end side P2 of the second elongated hole 122, so that the assisting device 1 shifts to the gear driving mode M3. Thereby, the relative position of the assisting gear 17 located on the distal end portion of the main shaft 13 with respect to the frame 14 is also changed from the position P1 to the position P2, and the assisting gear 17 engages with the driven gear 235 (specifically, the grasping shaft gear 2351) of the manipulator 2. By rotating the handle 11 in this state, the assisting device 1 can operate the forceps 211 of the manipulator 2. As illustrated in Fig. 9C and Fig. 11, in the neutral mode M1, when the handle 11 slides to the other end side of the second elongated hole 122, the main shaft 13 connected to the handle 11 slides to the other end side P3 of the second elongated hole 122, so that the assisting device 1 shifts to the gear driving mode M3. Thereby, the relative position of the assisting gear 17 located on the distal end portion of the main shaft 13 with respect to the frame 14 is also changed from the position P1 to the position P3, and the assisting gear 17 engages with the driven gear 235 (specifically, the bending shaft gear 2354) of the manipulator 2. By rotating the handle 11 in this state, the assisting device 1 can operate the second joint 214 of the manipulator 2. As described above, in the gear driving mode M3, when the user slides the handle 11 while the assisting device 1 is connected to the manipulator 2, one of the driven gears 235 of the manipulator 2 is selectively engaged with the assisting gear 17 of the assisting device 1 to make it possible to operate one of the end effectors 21. In the gear driving mode M3, from the state that the handle 11 and the main shaft 13 are located on one end side P2 of the second elongated hole 122, the handle 11 is slid to the other end side of the second elongated hole 122 without temporal stopping at the position P1 (neutral mode M1), so that the handle 11 and the main shaft 13 can be located on the other end side P3 of the second elongated hole 122, and vice versa.

As illustrated in Fig. 4, the assisting device 1 transitions to the washing mode M2 when the dial 12 is rotated from the first position illustrated in Fig. 10A to the second position illustrated in Fig. 10B in the neutral mode M1. The assisting device 1 returns to the neutral mode M1 when the handle 11 is pulled toward the proximal end side (Fig. 1: +X axis direction) or the side lock button 15 is pushed toward the frame 14 side in the washing mode M2. As illustrated in Fig. 4, the assisting device 1 transitions to the gear driving mode M3 when the handle 11 is moved to the end portion (Fig. 11: P2, P3) of the second elongated hole 122 of the dial 12 position in the neutral mode M1. The assisting device 1 returns to the neutral mode M1 when the handle 11 is moved to the center (Fig. 11: P1) of the second elongated hole 122 of the dial 12 in the gear driving mode M3.

As described above, the dial 12 of the assisting device 1 at the first position illustrated in Fig. 10A makes the positions of the notch 121 and the washing nozzle 19 different from each other, and makes the directions of the second elongated hole 122 and the first elongated hole 145 coincident with each other. The dial 12 of the assisting device 1 at the second position illustrated in Fig. 10B makes the positions of the notch 121 and the washing nozzle 19 coincident with each other, and makes the directions of the second elongated hole 122 and the first elongated hole 145 different from each other (in other words, the second elongated hole 122 and the first elongated hole 145 are oriented in different directions). As a result, the dial 12 of the assisting device 1 can functionally serves as a switching portion that allows switching between the washing mode M2 and the neutral mode M1 and switching between the gear driving mode M3 and the neutral mode M1, and meanwhile restricts switching between the gear driving mode 3 and the washing mode M2 not through the neutral mode M1.

The difference in the position between the notch 121 and the washing nozzle 19 means that the notch 121 of the dial 12 and the openings (the first proximal end opening 191 and the second proximal end opening 192) of the washing nozzle 19 do not overlap each other, and includes the aspects illustrated in Figs. 9A, 9C, and 9D, i.e. a case that the dial 12 closes at least a part of the opening of the washing nozzle 19 and therefore the syringe cannot be attached to the opening of the washing nozzle 19. The coincidence in the position between the notch 121 and the washing nozzle 19 means that the notch 121 of the dial 12 and the openings (the first proximal end opening 191 and the second proximal end opening 192) of the washing nozzle 19 overlap each other, and includes the aspect illustrated in Fig. 9B, i.e. a case that the dial 12 does not close the opening of the washing nozzle 19 and therefore the syringe can be attached to the opening of the washing nozzle 19.

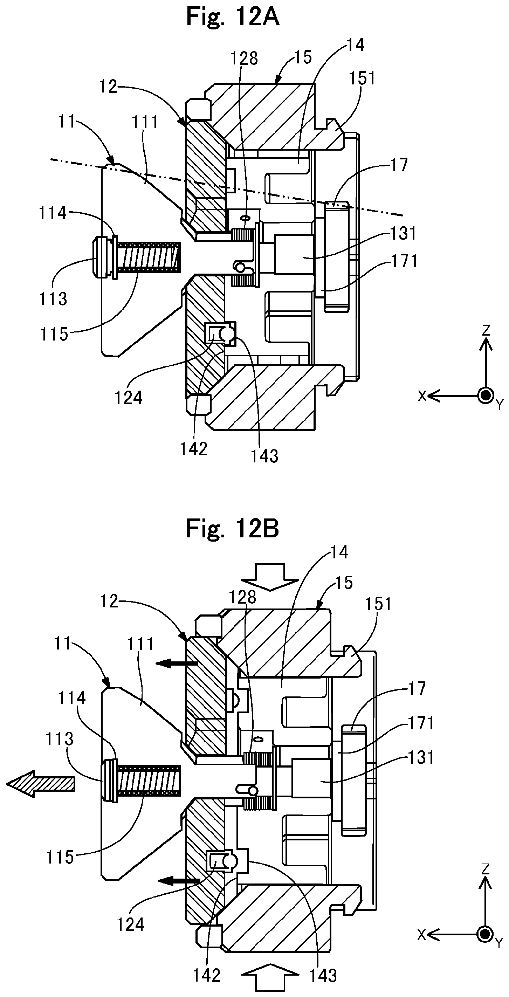

Figs. 12A and 12B are explanatory longitudinal sectional views of the assisting device 1. Fig. 12A is a longitudinal sectional view of the assisting device 1 in a normal state. The dial 12 is energized by a dial rotation spring 129 (see Fig. 7) mounted between the dial 12 and the frame 14 in a moving direction from the second position illustrated in Fig. 10B to the first position illustrated in Fig. 10A, i.e. a rotation direction from the second position to the first position (returning direction). The handle 11 is energized toward the dial 12 side by the mounted knob latch spring 115. Thus, in the normal state, automatic rotation of the dial 12 due to energization of the dial rotation spring 129 is suppressed by the force applied from the handle 11 to the dial 12 (the force pressing the dial 12 toward the frame 14 side). Herein, the normal state means a state that the handle 11 is not pulled and the side lock button 15 is not pressed down. As illustrated in Fig. 12A, the dial lock pin 124 disposed on the distal end-side surface of the dial 12 engages with the recess portion 143 disposed on the proximal end surface 142 of the frame 14 to maintain the state (the first position and the second position) of the dial 12.

Fig. 12B is a longitudinal sectional view of the assisting device 1 in a state that the assisting device 1 returns to the neutral mode M1 from the washing mode M2. In the washing mode M2, when the handle 11 is pulled toward the proximal end side as indicated by the hatched arrow, the force applied from the handle 11 toward the dial 12 (force that presses the dial 12 toward the frame 14 side) disappears. The dial 12 is energized toward the handle 11 side by the spring 128. As a result, the engagement between the dial lock pin 124 and the recess portion 143 is released, and the dial 12 automatically rotates from the second position illustrated in Fig. 10B to the first position illustrated in Fig. 10A and returns to the neutral mode M1 from the washing mode M2. In the washing mode M2, when the side lock button 15 is pushed toward the frame 14 side as indicated by the white blank arrow, the dial 12 is pressed from the side lock button 15 toward the handle 11 side in association with the movement of the side lock button 15 toward the frame 14 side, to disengage the dial lock pin 124 from the recess portion 143, so that the dial 12 automatically rotates from the second position to the first position and returns to the neutral mode M1 from the washing mode M2.

As illustrated in Fig. 11, no matter how much the grasping shaft gear 2351 and the bending shaft gear 2354 according to the first embodiment rotate in the D1 direction indicated by the solid line, no problem is caused, but it is preferable that they are not excessively rotated in the D2 direction indicated by the dashed line. Herein, the "excessively rotated" means that the torque applied to the handle 11 exceeds a predetermined level. Thus, the assisting device 1 according to the first embodiment includes a torque limiting mechanism. Specifically, in the gear driving mode M3, when the handle 11 and the main shaft 13 located at one end side P2 of the second elongated hole 122 are rotated in the direction indicated by the solid line arrow, the main shaft 13 does not deviate, and the assisting gear 17 can continue to transmit the rotational force to the grasping shaft gear 2351. On the other hand, when the handle 11 and the main shaft 13 are rotated in the direction indicated by the dashed line arrow, the main shaft 13 deviates (a phenomenon that the main shaft 13 is inclined with respect to the longitudinal direction of the assisting device 1, i.e. the X axis direction), so that the engagement between the assisting gear 17 and the grasping shaft gear 2351 is released, and the rotational force cannot be transmitted to the grasping shaft gear 2351. This is achieved by the balance between the force applied from the handle 11 toward the dial 12 (the force pressing the dial 12 toward the frame 14 side) and the deviation force of the main shaft 13. In the gear driving mode M3, similarly in a case that the handle 11 and the main shaft 13 are located on the other end side P3 of the second elongated hole 122, the main shaft 13 does not deviate when rotated in the direction indicated by the solid line arrow, and meanwhile the main shaft 13 deviates when rotated in the direction indicated by the dashed line arrow.

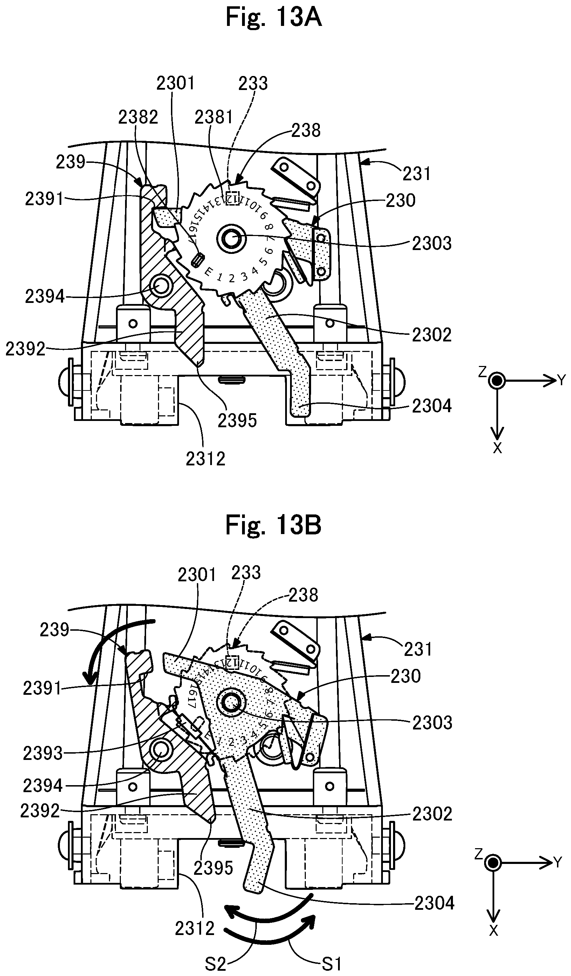

Figs. 13A and 13B are explanatory diagrams illustrating a configuration of the counter mechanism in the manipulator 2. Fig. 13A illustrates a first state of the counter mechanism. Fig. 13B illustrates a second state of the counter mechanism. Figs. 13A and 13B illustrate the manipulator 2 in a state that the lid 232 (see Fig. 8) is excluded. In Figs. 13A and 13B, for convenience of explanation, different types of hatching are given to some of the members, the configuration hiding the counter mechanism in the configuration of the manipulator main body 231, and the peripheral portion thereof are indicated by the dashed lines, and the window 233 of the lid 232 is indicated by the dashed lines. With reference to Figs. 13A and 13B, the (b) function of counting the uses of the manipulator 2 will be explained.

As illustrated in Figs. 13A and 13B, the manipulator 2 includes a mechanical counter mechanism. The manipulator 2 can be used multiple times by washing after each use. The counter mechanism counts the remaining uses of the manipulator 2, i.e. the washings of the manipulator 2. The counter mechanism includes a counter disk 238, a counter lever 230 (first member), and a lock lever 239 (second member). The counter disk 238 has a disk shape, i.e. a circular plate shape. The counter disk 238 is fixed to the manipulator main body 231 so as to be rotatable around a shaft 2303. On the main surface (the surface in the +Z axis direction) of the counter disk 238, a count number 2381 "E, 1, 2, ..., 16, 17" is provided. In the counter disk 238, only one count number is visible to the outside through the window 233 (dashed line) of the lid 232, and therefore the user can confirm the remaining uses of the manipulator 2. That means, the counter disk 238 and the window 233 functionally serve as an "indicating portion". Note that the "E" of the count number 2381 means that the number of the remaining uses of the manipulator 2 is zero. On the main surface of the counter disk 238, a protruding portion 2382 with a part of the main surface raised toward the lid 232 is formed at a position adjacent to the count number 2381 "E" (adjacent to a side opposite to the count number 2381 "1").

The counter lever 230 is fixed to the manipulator main body 231 so as to be rotatable around the shaft 2303 (i.e. coaxially with the counter disk 238). The counter lever 230 includes a first extending portion 2301 extending toward the lock lever 239 and a second extending portion 2302 extending toward the proximal end portion of the manipulator main body 231. As illustrated in Fig. 13B, when the counter lever 230 itself rotates to the one side S1, the counter lever 230 rotates the counter disk 238 by only one scale of the count number 2381. When the counter lever 230 itself rotates to the other side S2, the counter lever 230 does not rotate the counter disk 238. The counter lever 230 corresponds to the "first member".

The lock lever 239 is fixed to the manipulator main body 231 so as to be rotatable around a shaft 2394. The lock lever 239 has, on a part of the counter lever 230 side, a recess portion 2391 for engaging with the first extending portion 2301 of the counter lever 230. The lock lever 239 has an extending portion 2392 extending toward the proximal end portion of the manipulator main body 231. The lock lever 239 is configured such that the lock lever 239 and the counter lever 230 pull each other via a spring 2393. As illustrated in Fig. 13A, the lock lever 239 restricts the counter lever 230 to rotate to the other side S2 by engaging the first extending portion 2301 of the counter lever 230 with the recess portion 2391 of the lock lever 230. The lock lever 239 corresponds to the "second member".

That means, as illustrate in Fig. 13A, in the first state of the counter mechanism, the lock lever 239 (second member) and the counter lever 230 (first member) engage with each other, and the counter lever 230 rotates relatively to the one side S1. Thus, in the first state of the counter mechanism, an end portion 2304 of the counter lever 230 is accommodated in the manipulator main body 231. In this way, since the end portion 2304 is accommodated in the manipulator main body 231, there is no member located inside the notch 2312, and the surgery supporting robot is allowed to be connected to the manipulator 2 in the first state (see Fig. 8). As illustrated in Fig. 13B, in the second state of the counter mechanism, the lock lever 239 (second member) and the counter lever 230 (first member) do not engage with each other, and the counter lever 230 is pulled by the spring 2393 to rotate relatively to the other side S2. Thus, in the second state of the counter mechanism, the end portion 2304 of the counter lever 230 protrudes outside the manipulator main body 231. In this way, since the end portion 2304 protrudes outside the manipulator main body 231, the surgery supporting robot is prevented from being connected to the manipulator 2 in the second state due to the interference of the end portion 2304 located inside the notch 2312.

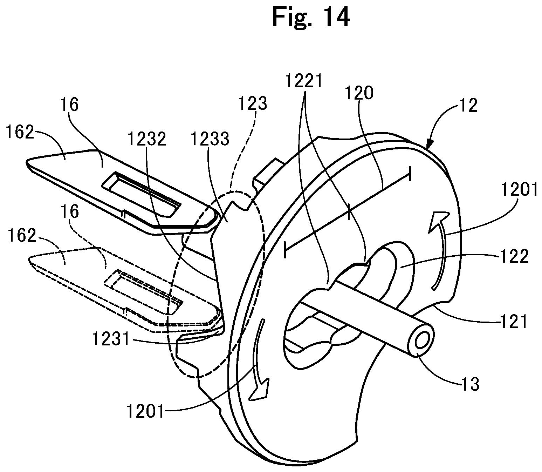

Fig. 14 is an explanatory diagram illustrating an actuator that cause the counter mechanism to operate. In Fig. 14, the dial 12, the pin 16, and the main shaft 13 in the assisting device 1 are exclusively illustrated. The state transition between the first state and the second state will be explained with reference to Fig. 14.

A cam structure portion 123 for operating the pin 16 is disposed on a peripheral edge portion of the dial 12 on the distal end side (the frame 14 side). The cam structure portion 123 is a hollow cylindrical cam having a recess portion 1231 with a smaller thickness of the dial 12 and a concaved distal end of the dial 12, a convex portion 1233 with a larger thickness of the dial 12 and a protruding distal end of the dial 12, and an inclined portion 1232 provided between the recess portion 1231 and the convex portion 1233. As described above, the pin 16 is energized toward the proximal end side (the handle 11 side) by the spring disposed on the elongated hole 161 of the pin 16. When the assisting device 1 is in the neutral mode M1 or the gear driving mode M3, the proximal end portion of the pin 16 (Fig. 14: dashed line) is accommodated in the recess portion 1231 so that the distal end portion 162 of the pin 16 is accommodated in the frame 14. On the other hand, when the assisting device 1 transitions from the neutral mode M1 to the washing mode M2, the proximal end of the pin 16 slides on the inclined portion 1232 and moves to the convex portion 1233 in association with the rotation of the dial 12, so that the pin 16 is pressed and accordingly the distal end portion 162 of the pin 16 protrudes from the distal end of the frame 14. When the pressing force from the cam structure portion 123 is released, the pin 16 is accommodated in the frame 14 again by the energization force of the spring in the elongated hole 161. In this way, when the pin 16 moves, the pin 16 slides within the through-hole provided on the frame 14, but the pin 16 is guided by the surface that demarcates the through-hole of the frame 14, so that the pin 16 can move without deviation.

As a result, when the assisting device 1 shifts to the washing mode M2, an end portion 2395 of the lock lever 239 (second member) is pushed to the distal end side by the distal end portion 162 protruding from the frame 14, the engagement between the lock lever 239 and the counter lever 230 (first member) is released, so that the counter lever 230 pulled by the spring 2393 rotates relatively to the other side S2. In other words, when the assisting device 1 transitions to the washing mode M2, the end portion 2395 of the lock lever 239 is pushed to the distal end side by the distal end portion 162 of the pin 16 protruding from the frame 14, and thereby the counter mechanism shifts from the first state illustrated in Fig. 13A to the second state illustrated in Fig. 13B. The "first operation" for shifting the counter mechanism from the first state to the second state may be an operation in which the end portion 2395 of the lock lever 239 (second member) is pushed to the distal end side of the manipulator 2 to release the engagement between the lock lever 239 and the counter lever 230 (first member) so that the counter lever 230 pulled by the spring 2393 is rotated relatively to the other side S2, i.e. an operation in which the dial 12 is rotated from the first position illustrated in Fig. 10A to the second position illustrated in Fig. 10B.

When the counter mechanism is in the second state illustrated in Fig. 13B, the user's fingers are hooked at the end portion 2304 of the counter lever 230 protruding outside the manipulator main body 231 to rotate the counter lever 230 to the one side S1, so that the counter mechanism shifts from the second state illustrated in Fig. 13B to the first state illustrated in Fig. 13A. At this time, the counter disk 238 rotates by only one scale of the count number 2381 in association with the rotation of the counter lever 230, and the numeric character presented to the outside through the window 233 is decremented (-1).

Thus, the second state refers to a state that the count can be decremented in the counter mechanism. The "second operation" for shifting the counter mechanism from the second state to the first state refers to an operation in which the user rotates the counter lever 230 to the one side S1.

In this way, in the counter mechanism according to the first embodiment, the shifts from the second state to the first state by the second operation are counted. In the counter mechanism according to the first embodiment, while the second operation can be manually performed by the user, the first operation requires the rotation operation of the dial 12, and therefore, the first operation is more difficult (hard to operate) than the second operation.

In the counter mechanism, when the shifts from the second state to the first state (i.e. the rotations of the counter disk 238) reach a predetermined count e.g. 17 times in Figs. 13A and 13B, the protruding portion 2382 of the counter disk 238 interferes with (abuts against) the projection provided on the inner surface of the lid 232 (the surface on the counter disk 238 side) on the edge of the window 233, thereby restricting further rotation of the counter disk 238 and the counter lever 230. As a result, the counter mechanism according to the first embodiment cannot shift to the first state illustrated in Fig. 13A when the shifts from the second state to the first state by the second operation reach a predetermined count. At this time, the count number 2381 presented to the outside from the window 233 is "E". That means, when the count number 2381 "E" is indicated on the window 233, the assisting device 1 can be attached to the manipulator 2 and washed in the washing mode M2 after use of the manipulator 2 but the counter lever 230 cannot rotate to the one side S1 (because the protruding portion 2382 of the counter disk 238 interferes with the projection provided on the edge of the window 233), and therefore the manipulator 2 cannot be attached to the robot arm for use. Although the predetermined count is set to 17 times in the example of Figs. 13A and 13B, the count may be predetermined as appropriate depending on the durability of the manipulator 2 against the sterilization process.

As illustrated in Fig. 14, in addition to the position indicator 120, a direction indicator 1201 that is an arrow indicating a rotational direction for rotating the dial 12 from the first position illustrated in Fig. 10A to the second position illustrated in Fig. 10B may be provided on the proximal end-side (handle 11 side) surface of the dial 12. On the peripheral edge portion of the second elongated hole 122 in the dial 12, raised portions 1221 may be formed, where the inner peripheral edge portion of the dial 12 raises toward the center in the longitudinal direction of the second elongated hole 122. The raised portions 1221 are provided on boundary portions between the positions P1, P2, and P3 of the handle 11 and the main shaft 13 in the neutral mode M3 and the gear driving mode M3.

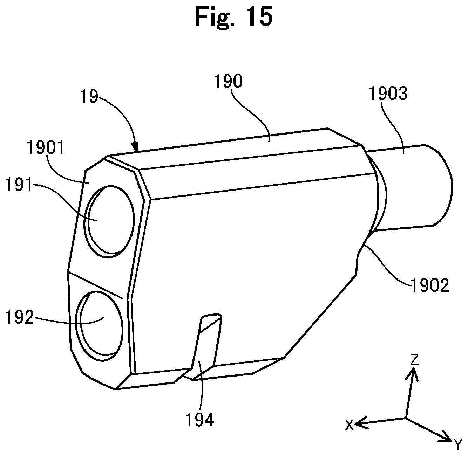

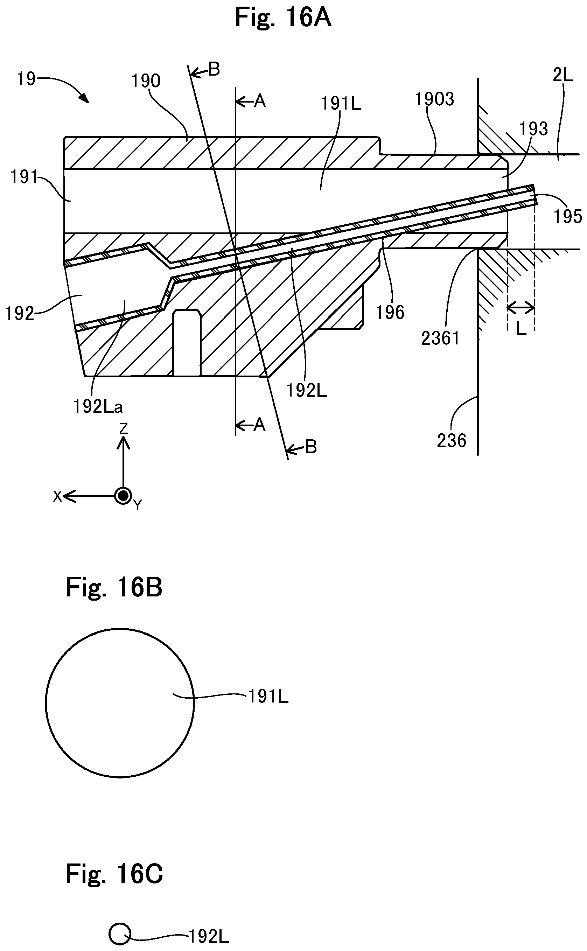

Fig. 15 and Figs. 16A to 16C are explanatory diagrams illustrating a configuration of the washing nozzle 19. Fig. 16A is a longitudinal sectional view of the washing nozzle 19 inserted into the manipulator 2. Fig. 16B is a transverse sectional view of a first flow passage 191L taken along line A-A in Fig. 16A. Fig. 16C is a transverse sectional view of a second flow passage 192L taken along line B-B in Fig. 16A. The line A-A is perpendicular to the extending direction of the first flow passage 191L, and the line B-B is perpendicular to the extending direction of the second flow passage 192L. Thus, Fig. 16B is a sectional view taken along a plane perpendicular to the extending direction of the first flow passage 191L, and Fig. 16C is a sectional view taken along a plane perpendicular to the extending direction of the second flow passage 192L. The configuration of the washing nozzle 19 will be explained with reference to Fig. 15 and Figs. 16A to 16C.

The washing nozzle 19 includes a nozzle main body 190. The nozzle main body 190 has a first surface 1901 located on the proximal end side and a second surface 1902 located on the distal end side. A protruding portion 1903 extends from the second surface 1902 of the nozzle main body 190 and further extends toward the distal end side. A notch 194 formed by cutting out a part of the nozzle main body 190 is formed on a lower part of the nozzle main body 190. Note that the notch 194 can be omitted. As illustrated in Fig. 16A, when the assisting device 1 is connected to the manipulator 2, the distal end portion of the nozzle main body 190 (specifically, the distal end portion of the protruding portion 1903) is inserted into the manipulator 2 from the washing port 2361 (opening) provided on the proximal end surface 236 of the manipulator main body 231. On the proximal end portion of the nozzle main body 190 (specifically, the first surface 1901), the first proximal end opening 191 and second proximal end opening 192 are provided. On the distal end portion of the nozzle main body 190 (specifically, the distal end of the protruding portion 1903), the first distal end opening 193 and the second distal end opening 195 are provided. When the assisting device 1 is connected to the manipulator 2, the first distal end opening 193 and the second distal end opening 195 communicate with the washing port 2361 of the manipulator 2.

The first flow passage 191L and the second flow passage 192L are formed inside the nozzle main body 190. The first flow passage 191L communicates with the first distal end opening 193 on the distal end and communicates with the first proximal end opening 191 on the proximal end. The second flow passage 192L communicates with the second distal end opening 195 on the distal end and communicates with the second proximal end opening 192 on the proximal end. As illustrated in Figs. 16B and 16C, the cross-sectional area of the second flow passage 192L is smaller than that of the first flow passage 191L. Herein, as illustrated in Fig. 16A, a part of the second flow passage 192L on the second proximal end opening 192 side is configured as an enlarged flow passage 192La having a large cross-sectional area so that a syringe as an external instrument can be inserted thereinto. Thus, the second flow passage 191L having a cross-sectional area smaller than of the first flow passage 192L illustrated in Fig. 16C means a part of the second flow passage 192L excluding the enlarged flow passage 192La. In the example of the figure, both the first flow passage 191L and the second flow passage 192L have a circular transverse sectional shape but may be formed into any shape other than the circular shape.

As illustrated in Fig. 16A, a second flow passage 192L is formed inside a second flow passage forming member 196. The second flow passage forming member 196 is arranged so as to incline with respect to the extending direction (X axis direction) of the nozzle main body 190 inside the nozzle main body 190. A part of the second flow passage forming member 196 on the distal end side protrudes into the first flow passage 191L from the inner peripheral surface of the first flow passage 191L. Thus, the distal end side of the nozzle main body 190 is configured such that the protruding portion 1903 and the second flow passage forming member 196 forms a double pipe, and a part of the second flow passage 192L on the distal end side is disposed inside the first flow passage 191L. The distal end portion of the second flow passage forming member 196 protrudes from the first distal end opening 193 to the distal end side (the manipulator 2 side, the flow passage 2L side in the manipulator 2). Thereby, the second distal end opening 195 on the distal end of the second flow passage 192L protrudes from the first distal end opening 193, and is located on the distal end side (the manipulator 2 side, the flow passage 2L side in the manipulator 2) with respect to the first distal end opening 193. A protrusion length L of the second distal end opening 195, i.e. a direct distance L between the second distal end opening 195 and the first distal end opening 193 may be determined as appropriate.

Under a condition that a passage resistance of the first flow passage 191L is defined as FR1, a passage resistance of the second flow passage 192L is defined as FR2, and a passage resistance of the flow passage 2L inside the manipulator 2 (i.e. the flow passage 2L communicating with the washing port 2361 in the manipulator 2) is defined as FR3, the relationship represented by Equation (1) below is established in the manipulator system 9 according to the first embodiment. That means, the passage resistance FR2 of the second flow passage 192L is higher than the passage resistance FR3 of the flow passage 2L inside the manipulator 2.

$$FR3 < FR2 \dots (1)$$

Furthermore, in the manipulator system 9, it is preferable that a relationship represented by Equation (2) below is established. That means, the passage resistance FR1 of the first flow passage 191L is lower than the passage resistance FR3 of the flow passage 2L inside the manipulator 2, and the passage resistance FR2 of the second flow passage 192L is higher than the passage resistance FR3 of the flow passage 2L inside the manipulator 2.

$$FR1 < FR3 < FR2 \dots (2)$$

The level relationship between the passage resistance FR1, the passage resistance FR2, and the passage resistance FR3, i.e. the relationships of Equations (1) and (2) can be confirmed by measuring volumes of water that flows out from the first proximal end opening 191, the second proximal end opening 192, and the forceps 211 when the first flow passage 191L, the second flow passage 192L, and the flow passage 2L of the manipulator 2 are filled with water from the first proximal end opening 191. The relationship between the passage resistance FR1 and the passage resistance FR2 is obviously FR1<FR2, from the size relationship of the cross-sectional areas between the first flow passage 191L and the second flow passage 192L.

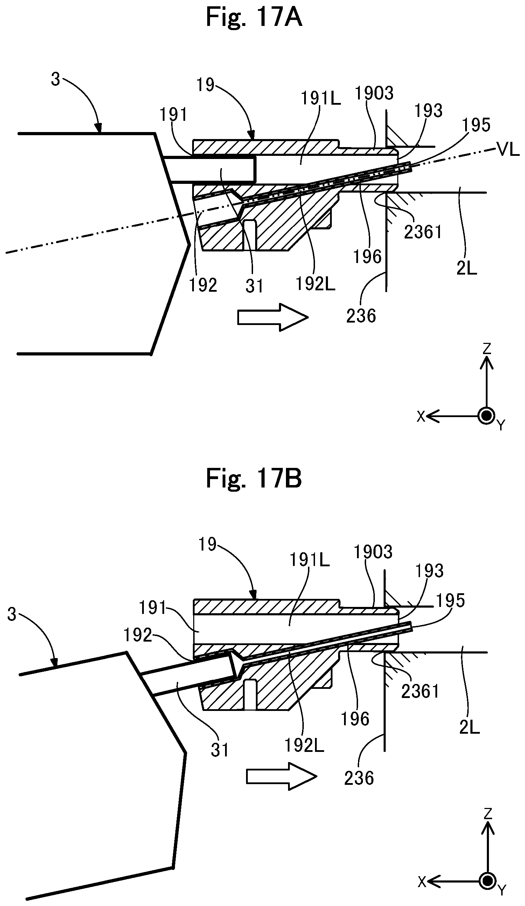

Figs. 17A and 17B are explanatory diagrams illustrating a method for using the washing nozzle 19. Fig. 17A illustrates a state that the washing fluid is injected into the flow passage 2L inside the manipulator 2. Fig. 17B illustrates a state that the washing fluid is discharged from the flow passage 2L inside the manipulator 2.

A case that the washing fluid is injected into the flow passage 2L to wash the inside of the main body device 23, the inside of the main shaft 22, and the end effector 21 will be explained with reference to Fig. 17A. In this case, the distal end portion of the syringe 3 filled with the washing fluid is inserted into the first flow passage 191L from the first proximal end opening 191. The washing fluid that has been fed from the syringe 3 by the insertion of the plunger into the syringe 3 flows out from the first distal end opening 193 through the first flow passage 191L, and flows into the flow passage 2L of the manipulator 2. When the washing fluid flows into the flow passage 2L, air in the flow passage 2L is pushed out from the second distal end opening 195 to the second flow passage 192L, and then discharged to the outside through the second proximal end opening 192. Thereby, the washing fluid can be smoothly injected into the flow passage 2L in the manipulator 2 without an excessive force.

A case that the washing fluid for filling the flow passage 2L in the manipulator 2 is discharged to the outside will be explained with reference to Fig. 17B. In this case, the distal end portion of the syringe 3 filled with air (or optionally another gas) is inserted into the second flow passage 192L (specifically, the enlarged flow passage 192La) from the second proximal end opening 192. Air that has been fed from the syringe 3 by the insertion of the plunger into the syringe 3 flows out from the second distal end opening 195 through the second flow passage 192L, and flows into the flow passage 2L of the manipulator 2. When air flows into the flow passage 2L, the washing fluid in the flow passage 2L is pushed from the first distal end opening 193 to the first flow passage 191L, and then discharged to the outside through the first proximal end opening 191. Thereby, the washing fluid can be smoothly discharged from the flow passage 2L in the manipulator 2 without an excessive force. That means, it can be said that the first flow passage 191L is for fluids and the second flow passage 192L is for gases (air).

As indicated by the white blank arrow in Fig. 17A, when the syringe 3 is pushed to the manipulator 2, i.e. when an external force is applied from the first proximal end opening 191, the position of the washing nozzle 19 in the assisting device 1 slides from the proximal end side toward the distal end side (i.e. toward the manipulator 2 side). Similarly, as indicated by the white blank arrow in Fig. 17B, when the syringe 3 is pushed to the manipulator 2, i.e. when an external force is applied from the second proximal end opening 192, the position of the washing nozzle 19 in the assisting device 1 slides from the proximal end side toward the distal end side (i.e. toward the manipulator 2 side). These configurations are achieved when the washing nozzle 19 is slidably fixed to the frame 14 of the assisting device 1. In this case, when an external force is applied from the first proximal end opening 191 or the second proximal end opening 192, the washing nozzle 19 slides along the X axis from the proximal end side toward the distal end side (i.e. toward the manipulator 2 side). For example, as illustrated in Fig. 17A, the first distal end opening 193 and the first proximal end opening 191 located on both ends of the first flow passage 191L are located on a same straight line along the sliding direction (X axis) of the washing nozzle 19. On the other hand, the second proximal end opening 192 located on the proximal end of the second flow passage 192L is located on a virtual line VL intersecting the sliding direction (X axis).

Each member constituting the assisting device 1 and each member constituting the manipulator 2 described above can be made of a well-known resin material or a well-known metal material. As described above, electronic components or the like are not used for the assisting device 1 and the manipulator 2, and the assisting device 1 and the manipulator 2 have a simple structure composed of combinations of members made of generally used materials, and therefore they can withstand wetting resulting from the washing process and a high temperature in the sterilization process.

As described above, in the manipulator system 9 according to the first embodiment, the manipulator assisting device 1 (manipulator assisting device main body) includes: the assisting gear that engages with the driven gears 235 when the assisting device 1 is connected to the manipulator 2; and the handle 11 that transmits a drive force to the assisting gear 17. Thus, after the assisting device 1 is connected to the proximal end portion of the manipulator 2, the handle 11 of the assisting device 1 is operated to drive the driven gears 235 of the manipulator 2 via the assisting gear 17, so that the end effector 21 can be operated. That means, according to the manipulator system 9, it is possible to provide the assisting device 1 that allows the end effector 21 of the manipulator 2 to operate with no medical device (surgery supporting robot) by operating the handle 11 of the assisting device 1. As a result, after the assisting device 1 is connected to the proximal end portion of the manipulator 2, the manipulator 2 is washed by operating the handle 11 of the assisting device 1, so that it is possible to wash the manipulator while the movable portions (forceps 211, second joint 214) of the end effector 21 take various postures. Furthermore, by operating the handle 11 of the assisting device 1 after connecting the assisting device 1 to the proximal end portion of the manipulator 2, the manipulator 2 can be removed from a body cavity of a patient after the movable portions (forceps 211, second joint 214) of the end effector 21 is brought into a safe shape for removing the manipulator from the body cavity of the patient (e.g. a state that the second joint 214 is straight and the forceps are closed).

In the manipulator system 9 according to the first embodiment, by sliding the handle 11 in a state that the assisting device 1 (manipulator assisting device main body) is connected to the manipulator 2, the assisting gear 17 can selectively engage with one of the plurality of driven gears 235 (specifically, the grasping shaft gear 2351 as the first driven gear and the bending shaft gear 2354 as the second driven gear) included in the manipulator 2. Consequently, when the manipulator 2 has the first and second driven gears 2351 and 2354 for causing the movable portions (forceps 211, second joint 214) of the end effector 21 to take various motions, the assisting device 1 can cause the assisting gear 17 to selectively engage with one of the first and second driven gears 2351 and 2354 to allow the movable portions (forceps 211, second joint 214) of the end effector 21 to selectively operate.

In the manipulator system 9 according to the first embodiment, since the assisting device 1 (manipulator assisting device main body) includes the washing nozzle 19, the washing fluid for washing the inside of the manipulator 2 can be easily fed to the flow passage 2L of the manipulator 2 through the washing nozzle 19 when the assisting device 1 is connected to the manipulator 2. In the manipulator system 9, since the assisting device 1 includes the pin 19 (actuator), the counter mechanism of the manipulator 2 can be switched to a countable state via the pin 16 (actuator) when the assisting device 1 is connected to the manipulator 2. As a result, the efficiency and the accuracy in washing the manipulator 2 can be improved. The count of washings of the manipulator 2 can be easily managed by using the counter mechanism.

In the manipulator system 9 according to the first embodiment, a user can cause the manipulator 2 to perform a desired operation by connecting the assisting device 1 (manipulator assisting device main body) to the manipulator 2 and switching between the gear driving mode M3 and the washing mode M2 via the dial 12 (switching portion). In the gear driving mode M3, the handle is allowed to slide and the washing nozzle 19 is restricted to be accessed, and in the washing mode M2, the handle 11 is restricted to slide and the washing nozzle 19 is allowed to be accessed. As a result, the assisting device 1 at each mode can prevent the user from performing an operation other than the operations allowed in each mode. That means, the user can be prevented from performing an erroneous operation.

In the manipulator system 9 according to the first embodiment, the assisting device 1 (manipulator assisting device main body) further has the neutral mode M1 in addition to the gear driving mode M3 and the washing mode M1. The dial 12 (switching portion) of the assisting device 1 allows the switching between the gear driving mode M3 and the neutral mode M1 and the switching between the washing mode M2 and the neutral mode M1, and meanwhile restricts the switching between the gear driving mode M3 and the washing mode M2 not through the neutral mode M1. Thereby, it is possible to suppress an erroneous operation that, during the operation of the manipulator 2 in the gear driving mode M3 in association with operation of the end effector 21, the assisting device 1 is erroneously switched to the washing mode M2 accompanied by decrement of the counter mechanism, and it is possible to improve the usability of the assisting device 1.

Furthermore, in the manipulator system 9 according to the first embodiment, the relative position of the assisting gear 17 with respect to the frame 14 can be changed by sliding the handle 11 of the assisting device 1 (manipulator assisting device main body) in the frame 14.

In the manipulator system 9 according to the first embodiment, for the assisting device 1 (manipulator assisting device main body), the disc-shaped dial 12 having the notch 121 is used as the switching portion, which is disposed between the handle 11 and the frame 14 and rotatable around the main shaft 13. Thereby, the dial 12 can restrict the washing nozzle 19 to be accessed by rotating the dial 12 to the first position where the notch 121 and the proximal end openings 191 and 192 of the washing nozzle 19 do not overlap with each other, and the dial 12 can allow the washing nozzle 19 to be accessed by rotating the dial 12 to the second position where the notch 121 and the proximal end openings 191 and 192 overlap with each other.

In the manipulator system 9 according to the first embodiment, the dial 12 used as the switching portion in the assisting device 1 (manipulator assisting device main body) has the second elongated hole 122 that is oriented in the same direction as the first elongated hole 145 of the frame 14 when the dial 12 is rotated to the first position. As a result, by rotating the dial 12 to the first position, the second elongated hole 122 of the dial 12 and the first elongated hole 145 of the frame 14 are oriented in the same direction, so that the dial 12 can allow the handle 11 to slide, and by rotating the dial 12 to the second position, the second elongated hole 122 of the dial 12 and the first elongated hole 145 of the frame 14 are oriented in different directions, so that the dial 12 can restrict the handle 11 to slide.

In the manipulator system 9 according to the first embodiment, on the peripheral edge portion of the second elongated hole 122 of the dial 12 in the assisting device 1 (manipulator assisting device main body), the raised portions 1221 are formed, where the peripheral edge portion raises toward the second elongated hole 122. Thus, the raised portions 1221 are formed between the positions P2, P3 at which the assisting gear 17 of the assisting device 1 engages with the driven gears 235 of the manipulator 2 (Fig. 11) and the position P1 at which they do not engage with each other (Fig. 11), so that the user can easily grasp the mechanism, and the handle 11 can be prevented from deviating between the engaging positions P2, P3 and the non-engaging position P1. That means, the handle 11 can be prevented from being located between the position P2 and the position P1 or between the position P3 and the position P1.

In the manipulator system 9 according to the first embodiment, when a torque exceeding a predetermined level is applied to the handle 11 of the assisting device 1 (manipulator assisting device main body), the main shaft 13 "deviates" in a direction inclined from the longitudinal direction of the assisting device 1 i.e. the x axis direction to cause the handle 11 to release the engagement between the assisting gear 17 and the driven gears 235. As a result, even when a torque exceeding a predetermined level is applied to the handle 11, it is possible to prevent the driven gears 235 from excessively rotating to suppress failure of the end effector 21 due to the excessive rotation of the driven gears 235.

In the manipulator system 9 according to the first embodiment, it is possible to provide the manipulator system 9 including: the manipulator 2 connected to a medical device (surgery supporting robot) and having the end effector 21 to be operated by the medical device; and the assisting device 1 that allows the end effector 21 of the manipulator 2 to operate with no medical device.

In the manipulator system 9 according to the first embodiment, when the assisting device 1 (manipulator assisting device main body) is connected to the manipulator 2 and shifts to the washing mode M2 by the rotation of the dial 12, the counter lever 230 (first member) rotates relatively to the other side S2, and the end portion 2304 of the counter lever 230 protrudes outside the manipulator main body 231. That means, once the assisting device 1 is connected to the manipulator 2 to shift to the washing mode M2, the counter mechanism can be switched to a countable state (decrementable state). When the assisting device 1 is detached from the manipulator 2 and the end portion 2304 of the counter lever 230 (first member) protruding outside the manipulator main body 231 is accommodated inside the manipulator main body 231 in order to attach the manipulator 2 to the medical device (surgery supporting robot), the counter lever 230 rotates relatively to the one side S1, and the counter disk 238 rotates to cause the counter mechanism to count (decrement). That means, once the end portion 2304 of the counter lever 230 protruding outward is accommodated inside the manipulator main body 231, the counter mechanism can count (decrement) automatically.

In the manipulator system 9 according to the first embodiment, the counter mechanism of the manipulator 2 is subjected to the first operation to shift from the first state illustrated in Fig. 13A to the second state illustrated in Fig. 13B, and subjected to the second operation after the shifting to the second state to shift from the second state illustrated in Fig. 13B to the first state illustrated in Fig. 13A. Then, the counter mechanism of the manipulator 2 counts the shifts from the second state to the first state by the second operation. In the manipulator system 9 according to the first embodiment, in a situation of washing the manipulator 2, the first operation is executed by shifting the assisting device 1 to the washing mode M2 while the assisting device 1 is connected to the manipulator 2, but the second operation is not executed. This is because, even if the end portion 2304 of the counter lever 230 protrudes outside the manipulator main body 231 by execution of the first operation, the end portion 2304 of the counter lever 230 is located inside the assisting device 1, and therefore the second operation cannot be executed unless the assisting device 1 is detached. Thus, duplicated counting due to erroneous operation of the counter mechanism during the washing of the manipulator 2 can be prevented. On the other hand, in a situation of using the manipulator 2, since the manipulator 2 is attached to the medical device (surgery supporting robot) in a state that the assisting device 1 has been detached and the first operation has been executed (the end portion 2304 of the counter lever 230 protrudes outside the manipulator main body 231), the second operation needs to be executed. Thus, the counter mechanism can reliably perform the counter, so that the count number can be prevented from being skipped. That means, the counter mechanism has the first state of impossible counting and the second state of possible counting, and counts only the shifts from the second state to the first state, to makes it possible to prevent miscounting. By using such a counter mechanism for counting the uses of the manipulator 2, the uses of the manipulator 2 can be counted with high accuracy.

In the manipulator system 9 according to the first embodiment, the manipulator has a mechanical structure composed of the counter disk 238, the counter lever 230 (first member), and the lock lever 239 (second member), in which the counter disk is rotated by rotating the counter lever 230 (first member) of the counter mechanism relatively to the one side S1 during the shifting from the second state to the first state, so that the shifts can be automatically counted (decremented).

In the manipulator system 9 according to the first embodiment, when the counter mechanism of the manipulator 2 is in the first state illustrated in Fig. 13A, the end portion 2304 of the counter lever 230 (first member) is accommodated in the manipulator main body 231, and when the counter mechanism is in the second state illustrated in Fig. 13B, the end portion 2304 of the counter lever 230 (first member) protrudes outside the manipulator main body 231, and therefore the user can grasp the current state of the manipulator 2 (whether the first state or the second state, i.e. whether before or after the counter) at first sight. In the manipulator system 9, when the counter mechanism is in the second state illustrated in Fig. 13B, the end portion 2304 of the counter lever 230 (first member) protrudes outside the manipulator main body 231 to prevent the proximal end portion of the manipulator 2 from being connected to the medical device, and therefore the count of the transitions i.e. the shifts from the second state to the first state can be prevented from being missed to be decremented, in other words, the shifts can be prevented from being missed to be counted.

In the manipulator system 9 according to the first embodiment, the counter mechanism of the manipulator 2 inhibits the transition from the second state to the first state when the count of the transitions from the second state to the first state reaches the predetermined count. Herein, in the second state illustrated in Fig. 13B, since the end portion 2304 of the counter lever 230 (first member) protrudes outside the manipulator main body 231 to prevent the proximal end portion of the manipulator 2 from being connected to the medical device, the user can be prevented from erroneously attaching the manipulator 2 that has been used over an upper limit count of the shifts from the second state to the first state (i.e. the count of the uses of the manipulator 2) to the medical device and reusing it.

In the manipulator system 9 according to the first embodiment, since the counter mechanism of the manipulator 2 has the window 233 and the counter disk 238 as the indicating portions for indicating the count of the shifts, the user can easily confirm the count of the shifts from the second state to the first state (i.e. the count of the uses of the manipulator 2) by checking the indicating portions 233 and 238. As a result, usability of the manipulator 2 can be improved.

In the manipulator system 9 according to the first embodiment, the washing nozzle 19 of the assisting device 1 (manipulator assisting device main body) includes the first flow passage 191L and the second flow passage 192L having a cross-sectional area smaller than of the first flow passage 191L, the first flow passage 191L has the distal end communicating with the first distal end opening 193 and the proximal end communicating with the first proximal end opening 191, and second flow passage 192L has the distal end communicating with the second distal end opening 195 and the proximal end communicating with the second proximal end opening 192. Thus, when the washing fluid for washing the inside of the manipulator 2 is injected into the manipulator 2 from the syringe 3 as an external instrument, the syringe 3 is inserted into the first proximal end opening 191, and the washing fluid is injected into the manipulator 2 from the first flow passage 191L through the washing port 2361 of the manipulator 2, so that air inside the manipulator 2 can be released to the outside through the second distal end opening 195, the second flow passage 192L, and the second proximal end opening 192. As a result, the injection of the washing fluid into the manipulator 2 is facilitated. When the washing fluid is discharged after the washing of the manipulator 2, the syringe 3 is inserted into the second proximal end opening 192, and air is injected into the manipulator 2 from the second flow passage 192L through the washing port 2361 of the manipulator 2, so that the washing fluid inside the manipulator 2 can be discharged to the outside through the first distal end opening 193, the first flow passage 191L, and the first proximal end opening 191. As a result, the manipulator 2 can be efficiently washed by using the washing nozzle 19.

In the manipulator system 9 according to the first embodiment, since the second distal end opening 195 of the washing nozzle 19 protrudes from the first distal end opening 193 and is located on the distal end side with respect to the first distal end opening 193, it is easier to discharge air inside the manipulator 2 during the injection of the washing fluid and to inject air into the manipulator 2 during the discharge of the washing fluid.

In the manipulator system 9 according to the first embodiment, since the passage resistance FR2 of the second flow passage 192L of the washing nozzle 19 is higher than the passage resistance FR3 of the flow passage 2L included in the manipulator 2 and communicating with the washing port 2361 of the manipulator 2 (i.e. the passage resistance FR3 of the flow passage inside the manipulator), it is easier to discharge air inside the manipulator 2 during the injection of the washing fluid and to inject air into the manipulator 2 during the discharge of the washing fluid.

In the manipulator system 9 according to the first embodiment, it is possible to provide the assisting device 1 including the washing nozzle 19 capable of efficiently washing the manipulator 2.

In the manipulator system 9 according to the first embodiment, the washing nozzle 19 of the assisting device 1 (manipulator assisting device main body) is fixed to the frame 14 of the assisting device 1 so as to be slidable from the proximal end side toward the distal end side when an external force is applied from the first proximal end opening 191 or the second proximal end opening 192. Thereby, the external instrument 3 inserted into the first proximal end opening 191 or the second proximal end opening 192 is pushed toward the distal end side, so that the distal end portion of the washing nozzle 19 can be inserted more deeply into the washing port 2361 of the manipulator 2 to make more certain of the fixation between the washing nozzle 19 and the manipulator 2. As a result, operability of the washing fluid during the injection and the discharge can be improved.

In the manipulator system 9 according to the first embodiment, since the first distal end opening 193 and the first proximal end opening 191 of the washing nozzle 19 are arranged on the same straight line along the sliding direction of the washing nozzle 19, the washing fluid can be smoothly injected and discharged through the first distal end opening 193, the first flow passage 191L, and the first proximal end opening 191.

<Second Embodiment>

In the second embodiment, a configuration in which the assisting gear 17 engages with only one driven gear 235 in the gear driving mode M3 will be explained. The manipulator system 9A according to the second embodiment includes an assisting device 1A instead of the assisting device 1, and a manipulator 2A instead of the manipulator 2.

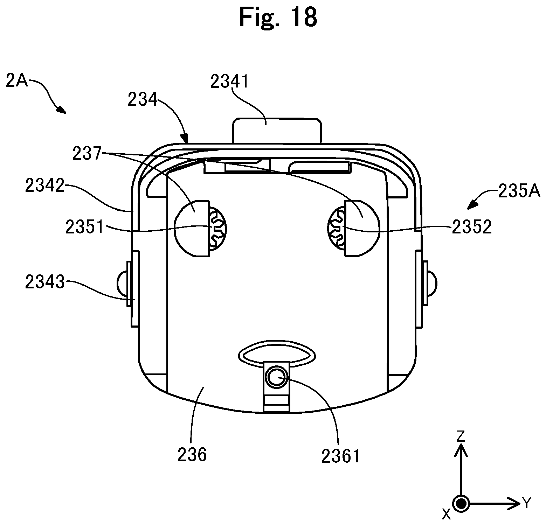

Fig. 18 is an explanatory diagram illustrating a configuration of the manipulator 2S according to the second embodiment. The manipulator 2A according to the second embodiment includes driven gears 235A instead of the driven gears 235. The driven gears 235A include the grasping shaft gear 2351 and the main shaft gear 2352 but does not have the distal end rotation shaft gear 2353 and the bending shaft gear 2354 described in the first embodiment. Thus, in the end effector 21 of the manipulator 2A, the forceps 211 are openable/closable and the main shaft 22 is rotatable, but the forceps 211 are not rotatable, and the second joint 214 is absent.

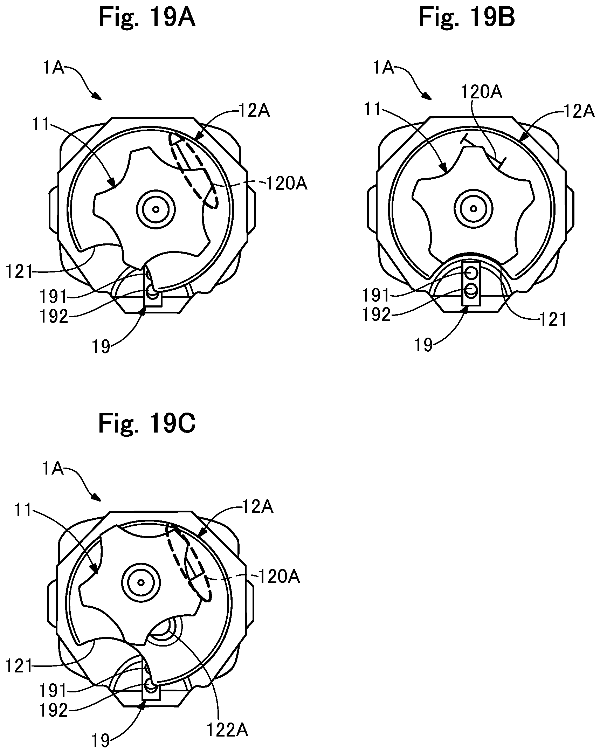

Figs. 19A to 19C are diagrams illustrating a state of the assisting device 1A in each mode according to the second embodiment. The assisting device 1A according to the second embodiment includes a dial 12A instead of the dial 12. The dial 12A includes a position indicator 120A instead of the position indicator 120, and a second elongated hole 122A instead of the second elongated hole 122. The position indicator 120A and the second elongated hole 122A have only portions corresponding to the center P1 and the one end side P2 described in the first embodiment. In other words, the position indicator 120A and the second elongated hole 122A do not have the portions corresponding to the other side P3 described in the first embodiment. Thus, as illustrated in Fig. 19C, in the gear driving mode M3, when the handle 11 is slid to one end side of the second elongated hole 122, the main shaft 13 connected to the handle 11 slides to the one end side P2 of the second elongated hole 122. As a result, the assisting gear 17 engages with the driven gears 235 (specifically, the grasping shaft gear 2351) of the manipulator 2A. By rotating the handle 11 in this state, the assisting device 1A can operate the forceps 211 of the manipulator 2A.

As described above, the configurations of the assisting device 1A and the manipulator 2A can be variously changed, and the assisting gear 17 may be configured to engage with only one driven gear 235 (grasping shaft gear 2351) in the gear driving mode M3. In the second embodiment, a configuration in which the bending shaft gear 2354 is omitted and the assisting gear 17 engages with the grasping shaft gear 2351 in the gear driving mode M3 is described as an example. However, it is allowed to adopt a configuration in which the grasping shaft gear 2351 is omitted and the assisting gear 17 engages with the bending shaft gear 2354 in the gear driving mode M3. The manipulator system 9A according to the second embodiment as described above can also exhibit the same effects as in the first embodiment described above.

<Third Embodiment>

In the third embodiment, a configuration in which the assisting gear 17 can selectively engage with all driven gears 235 in the gear driving mode M3 will be explained. A manipulator system 9B according to the third embodiment includes an assisting device 1B instead of the assisting device 1. The configuration of the manipulator 2 is the same as in the first embodiment.

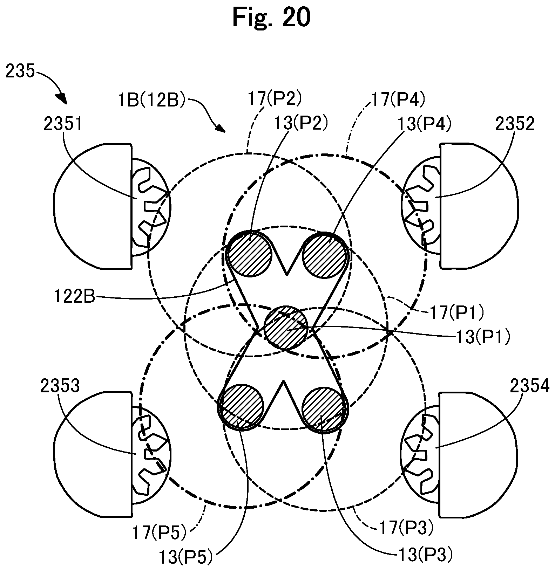

Fig. 20 is a diagram illustrating a relationship between the assisting gear 17 and the driven gears 235 in each mode according to the third embodiment. The assisting device 1B includes a dial 12B instead of the dial 12. The dial 12B has a second elongated hole 122B instead of the second elongated hole 122. The second elongated hole 122B has an X shape with two elongated holes intersecting with each other. The second elongated hole 122B has, in addition to the positions P1 to P3 described in the first embodiment, an end portion P4 located on the main shaft gear 2352 side with respect to the center P1, and an end portion P5 located on the distal end rotation shaft gear 2353 side with respect to the center P1.

In the gear driving mode M3, when the handle 11 is slid to the end portion P4 of the second elongated hole 122B, the main shaft 13 connected to the handle 11 slides to the end portion P4 of the second elongated hole 122B. Thereby, the relative position of the assisting gear 17 located on the distal end portion of the main shaft 13 with respect to the frame 14 is also changed from the position P1 to the position P4, and the assisting gear 17 engages with the main shaft gear 2352 of the manipulator 2. By rotating the handle 11 in this state, the assisting device 1B can rotate the main shaft 22 of the manipulator 2. Similarly, in the gear driving mode M3, when the handle 11 is slid to the end portion P5 of the second elongated hole 122B, the main shaft 13 connected to the handle 11 slides to the end portion P5 of the second elongated hole 122B. Thereby, the relative position of the assisting gear 17 located on the distal end portion of the main shaft 13 with respect to the frame 14 is also changed from the position P1 to the position P5, and the assisting gear 17 engages with the distal end rotation shaft gear 2353 of the manipulator 2. By rotating the handle 11 in this state, the assisting device 1B can rotate the forceps 211 of the manipulator 2.

As described above, the configuration of the assisting device 1B can be variously changed, and the assisting gear 17 may be configured to be selectively engageable with all of the driven gears 235. The assisting device 1B may be configured such that the assisting gear 17 is engaged with any three gears of the driven gears 235. In this case, the shape of the second elongated hole 122B of the dial 12B should be changed. The manipulator system 9B according to the third embodiment as described above can also exhibit the same effects as in the first embodiment described above.

<Fourth Embodiment>

In the fourth embodiment, a manipulator system 9C in which a part of the components described in the first embodiment is omitted will be explained. The manipulator system 9C according to the fourth embodiment includes an assisting device 1C instead of the assisting device 1, and a manipulator 2C instead of the manipulator 2.

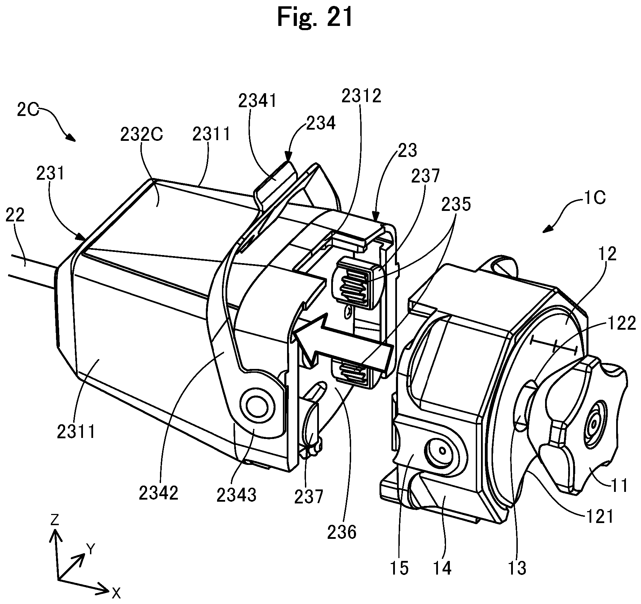

Fig. 21 is an explanatory diagram illustrating the assisting device 1C and the manipulator 2C according to the fourth embodiment. The assisting device 1C does not include the washing nozzle 19 explained in the first embodiment. Thus, the assisting device 1C does not transition to the washing mode M2 among the operation modes explained in Fig. 4. Thus, the notch 121 of the dial 12 may be omitted. The manipulator 2C includes a lid 232C instead of the lid 232. The lid 232C does not have the window 233 for indicating the count number 2381 of the counter disk 238. Accordingly, the remaining uses of the manipulator 2C cannot be confirmed from the outside. However, inside the manipulator 2C, the remaining uses are counted by the counter mechanism described in the first embodiment.

In this way, the configuration of the manipulator system 9C can be variously changed, and a part of the components described in the first embodiment, such as the washing nozzle 19 and the window 233, may be omitted. The above-described omissible configuration is merely an example, and other components not illustrated may be omitted. For example, the assisting device 1C may be configured such that the knob latch spring 115 or the dial rotation spring 129 is omitted, and the handle 11 or the dial 12 is not energized. For example, the manipulator 2C may be configured such that the protruding portion 2382 of the counter disk 238 is omitted so as not to inhibit the transitions from the second state to the first state over a predetermined count. For example, the lever 234 may be omitted in the manipulator 2C. The manipulator system 9C according to the fourth embodiment as described above can also exhibit the same effects as in the first embodiment described above.

<Fifth Embodiment>

In the fifth embodiment, a configuration that allows a state transition not through the neutral mode M1 will be explained. A manipulator system 9D according to the fifth embodiment includes an assisting device 1D instead of the assisting device 1. The configuration of the manipulator 2 is the same as in the first embodiment.

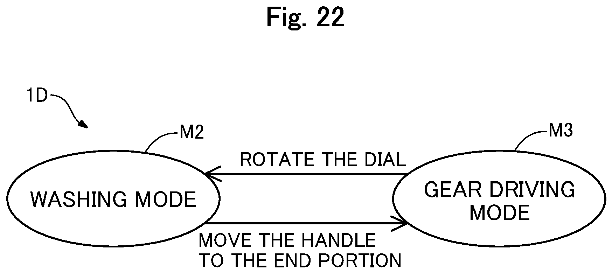

Fig. 22 is an explanatory diagram illustrating the state transition of the assisting device 1D according to the fifth embodiment. In the assisting device 1D, even if the dial 12 is located at the first position illustrated in Fig. 10A, the dial 12 does not close the openings (the first proximal end opening 191 and the second proximal end opening 192) of the washing nozzle 19. In the assisting device 1D, the hole formed on the dial 12, through which the main shaft 13 passes, is not the second elongated hole 122 described in the first embodiment, but is a circular hole through which the handle 11 can slide regardless of whether the dial 12 is in the first state or the second state. In the assisting device 1D, the knob latch spring 115 and the dial rotation spring 129 are omitted, and the handle 11 and the dial 12 are not energized. As a result, as illustrated in Fig. 22, the assisting device 1D can be directly switched between the washing mode M2 and the gear driving mode M3 not through the neutral mode M1.

As described above, the configuration of the manipulator system 9D can be variously changed, and the state transition of the assisting device 1D not through the neutral mode M1 is allowed. The manipulator system 9D according to the fifth embodiment as described above can also exhibit the same effects as in the first embodiment described above.

<Sixth Embodiment>

In the sixth embodiment, a configuration including a washing nozzle 19E different from the washing nozzle in the first embodiment will be explained. A manipulator system 9E according to the sixth embodiment includes an assisting device 1E instead of the assisting device 1. The configuration of the manipulator 2 is the same as in the first embodiment.

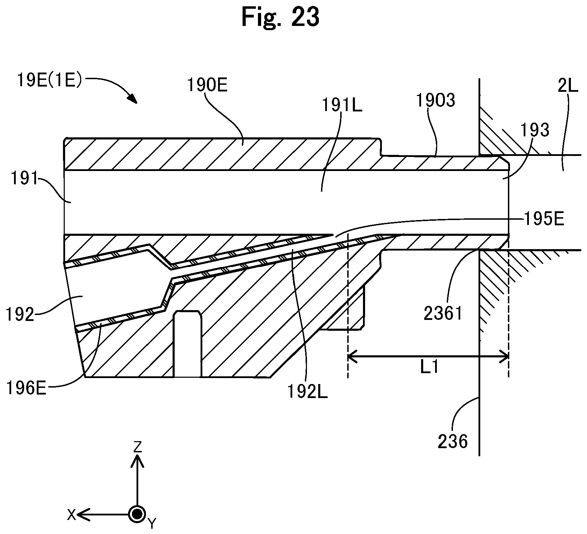

Fig. 23 is an explanatory diagram illustrating a configuration of the washing nozzle 19E according to the sixth embodiment. The assisting device 1E includes the washing nozzle 19E illustrated in Fig. 23. In the washing nozzle 19E, only the first distal end opening 193 is provided on a distal end portion of a nozzle main body 190E. When the assisting device 1E is connected to the manipulator 2, the first distal end opening 193 communicates with the washing port 2361 of the manipulator 2. A second flow passage forming member 196E is arranged to incline with respect to the extending direction (X axis direction) of the nozzle main body 190E inside the nozzle main body 190E. The distal end of the second flow passage forming member 196E is located on the inner peripheral surface of the first flow passage 191L, and the distal end portion of the second flow passage forming member 196E does not protrude into the first flow passage 191L. Thus, a second distal end opening 195E provided on the distal end of the second flow passage 192L is located on the inner peripheral surface of the first flow passage 191L. In other words, the first flow passage 191L and the second flow passage 192L merge together on the second distal end opening 195E. A direct distance L between the center of the second distal end opening 195E and the first distal end opening 193 may be determined as appropriate, but is preferably as short as possible (a small value).

As described above, the configuration of the washing nozzle 19E can be variously changed, and the second distal end opening 195E may be provided on the inner peripheral surface of the first flow passage 191L. The manipulator system 9E according to the sixth embodiment as described above can also exhibit the same effects as in the first embodiment described above. In the configuration according to the sixth embodiment, the second distal end opening 195E of the washing nozzle 19E is provided on the inner peripheral surface of the first flow passage 191L, and the first flow passage 191L and the second flow passage 192L merge together on the second distal end opening 195E. Thus, the washing nozzle 19E capable of efficiently washing the manipulator 2 and the assisting device 1E can be provided with a configuration in which the second distal end opening 195E is accommodated in the first flow passage 191L.

<Seventh Embodiment>

In the seventh embodiment, a configuration including a washing nozzle 19F different from the washing nozzle in the first embodiment will be explained. A manipulator system 9F according to the seventh embodiment includes an assisting device 1F instead of the assisting device 1. The configuration of the manipulator 2 is the same as in the first embodiment.

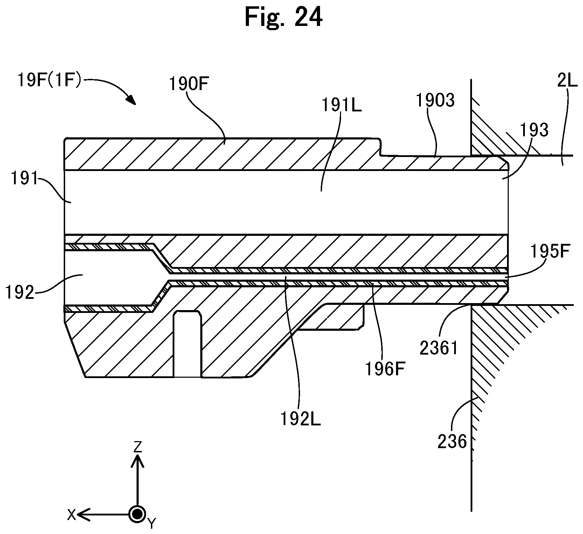

Fig. 24 is an explanatory diagram illustrating a configuration of the washing nozzle 19F according to the seventh embodiment. The assisting device 1F includes the washing nozzle 19F illustrated in Fig. 24. In the washing nozzle 19F, on a distal end portion of a nozzle main body 190F (specifically, the distal end of the protruding portion 1903), the first distal end opening 193 and a second distal end opening 195F are provided at different positions. As illustrated in Fit. 24, the first distal end opening 193 and the second distal end opening 195F are arranged apart from each other in the Z axis direction on the distal end surface of the protruding portion 1903. When the assisting device 1F is connected to the manipulator 2, the first distal end opening 193 and the second distal end opening 195F individually communicate with the washing port 2361 of the manipulator 2. A second flow passage forming member 196F is arranged in parallel with the extending direction (X axis direction) of the nozzle main body 190E inside the nozzle main body 190F.

As described above, the configuration of the washing nozzle 19F can be variously changed, and the first distal end opening 193 and the second distal end opening 195F may be arranged apart from each other at different positions. The manipulator system 9F according to the seventh embodiment as described above can also exhibit the same effects as in the first embodiment described above. In the configuration according to the seventh embodiment, the second distal end opening 195F of the washing nozzle 19F is provided at a position different from the position of the first distal end opening on the distal end portion of the nozzle main body 190F. Thus, the washing nozzle 19F capable of efficiently washing the manipulator 2 and the assisting device 1F can be easily manufactured.

<Modification Examples of Embodiments>

The disclosed embodiments are not limited to the above-described embodiments and may be implemented in various modes without departing from the gist thereof, and for example, the following modifications are also possible.

[Modification Example 1]

In the first to seventh embodiments described above, configuration examples of the manipulator systems 9, and 9A to 9F were described. However, the configuration of the manipulator system 9 can be variously changed. For example, the manipulator system 9 may further include other devices different from the assisting devices 1, and 1A to 1F and the manipulators 2, 2A, and 2C. For example, only some of the components of the manipulator system 9 may be used. Specifically, the manipulator system 9 may consist only of the assisting device 1, 1A, 1B, 1C, 1D, 1E, or 1F, or consist only of the manipulator 2, 2A, or 2C, or consist only of the washing nozzle 19, 19E, or 19F.

[Modification Example 2]

In the first to seventh embodiments described above, configuration examples of the assisting devices 1, and 1A to 1F were described. However, the configuration of the assisting device 1 can be variously modified. For example, the shapes of the handle 11, the dial 12, the main shaft 13, the frame 14, the side lock buttons 15, the pin 16, the assisting gear 17, the protruding portion 18, and the washing nozzle 19 may be changed as appropriate. For example, the position indicator 120 and the direction indicator 1201 of the dial 12 (switching portion) may be omitted. For example, the pin 16 (actuator) may be omitted. In this case, the cam structure portion 123 of the dial 12 for causing the pin 16 to operate can also be omitted. For example, although the counter mechanism has been described to decrement the count, the times of uses may be measured (counted) by incrementation. For example, the washing nozzle 19 may be configured to be fixed to the frame 14 without sliding when an external force is applied from the first proximal end opening 191 or the second proximal end opening 192. For example, a torque limit mechanism may be provided by a method other than the deviation of the main shaft 13. In this case, the handle 11 and the main shaft 13 may be connected via an O-ring made of an elastic material so that the handle 11 spins free against the main shaft 13 when the torque applied to the handle 11 exceeds a predetermined level.

[Modification Example 3]

In the first to seventh embodiments described above, configuration examples of the manipulators 2, 2A, 2C were described. However, the configuration of the manipulator 2 can be variously changed. For example, the movable portions included in the end effector 21 can be variously changed, and at least a part of the forceps 211, the first joint 212, the first extending portion 213, the second joint 214, and the second extending portion 215 may be omitted. Instead of the forceps 211, another surgical instrument (e.g. a laser scalpel) may be provided. For example, the movable portions of the end effector 21 may be replaceable. For example, the shapes of the various portions (the manipulator main body 231, the lid 232, the window 233, the lever 234, the driven gears 235, the gear covers 237, etc.) constituting the main body device 23 may be changed as appropriate. For example, the lid 232 may not be openable and closable, and for example, the gear covers 237 may be omitted. For example, the counter mechanism may be used for counting operations other than the washings of the manipulator 2. For example, the manipulator 2 need not include the counter mechanism. For example, the counter lever 230 of the manipulator 2 does not include the end portion 2304, and the end portion 2304 need not protrude outside the manipulator main body 231 in the second state of the counter mechanism.

[Modification Example 4]

The configurations of the manipulator systems 9, and 9A to 9B according to the first to seventh embodiments and the configurations of the manipulator systems 9, and 9A to 9F according to the modification examples 1 to 3 described above may be combined as appropriate. For example, in the manipulator systems 9A and 9B that perform the operations of the gear driving mode M3 described in the second embodiment and the gear driving mode M3 described in the third embodiment, the components described in the fourth embodiment and the neutral mode M1 described in the fifth embodiment may be omitted, and the washing nozzles 19E and 19F described in the sixth and seventh embodiments may be provided.

The aspects of the disclosed embodiments have been described above on the basis of the embodiments and modification examples, however, the embodiments of the aspects described above are intended to facilitate understanding of the aspects, and are not intended to limit the aspects. The aspects may be modified and improved without departing from the gist and the scope of claims and includes equivalents thereof. Unless the technical features are described as essential in the present specification, the technical features may be deleted as appropriate.

DESCRIPTION OF REFERENCE NUMERALS

1, and 1A to 1F ... Assisting device
2, 2A, and 2C ... Manipulator
3 ... External instrument, Syringe
9, and 9A to 9F ... Manipulator system
11 ... Handle
12, 12A, and 12B ... Dial (switching portion)
13 ... Main shaft
14 ... Frame (main body portion)
15 ... Side lock button
16 ... Pin (actuator)
17 ... Assisting gear
18 ... Protruding portion
19, 19E, and 19F ... Washing nozzle
21 ... End effector
22 ... Main shaft
23 ... Main body device
111 ... Grip portion
112 ... Extending portion
114 ... Washer
120 and 120A ... Position indicator
121 ... Notch
122, 122A, and 122B ... Second elongated hole
123 ... Cam structure portion
124 ... Dial lock pin
131 ... Bearing
141 ... Flange
142 ... Proximal end surface
143 ... Recess portion
144 ... Accommodation portion
145 ... First elongated hole
151 ... Claw
152 ... Shaft
161 ... Elongated hole
162 ... Distal end portion
171 ... Washer
190, 190E, and 190F ... Nozzle main body
191 ... First proximal end opening
191L ... First flow passage
192 ... Second proximal end opening
191L ... Second flow passage
192La ... Enlarged flow passage
193 ... First distal end opening
194 ... Notch
195, 195E, and 195F ... Second distal end opening
196, 196E, and 196F ... Second flow passage forming member

211 ... Forceps
212 ... First joint
213 ... First extending portion
214 ... Second joint
215 ... Second extending portion
230 ... Counter lever (first member)
231 ... Manipulator main body
232 and 232C ... Lid
233 ... Window (indicating portion)
234 ... Lever
235 and 235A ... Driven gear
236 ... Proximal end surface
237 ... Gear cover
238 ... Counter disk (indicating portion)
239 ... Lock lever (second member)
1201 ... Direction indicator
1221 ... Raised portion
1231 ... Recess portion
1232 ... Inclined portion
1233 ... Convex portion
1901 ... First surface
1902 ... Second surface
1903 ... Protruding portion
2301 ... First extending portion
2302 ... Second extending portion
2303 ... Shaft
2304 ... End portion
2311 ... Side surface
2341 ... Protruding portion
2342 ... Arm
2343 ... End portion
2351 ... Grasping shaft gear
2352 ... Main shaft gear
2353 ... Distal end rotation shaft gear
2354 ... Bending shaft gear
2361 ... Washing port
2381 ... Count number
2382 ... Protruding portion
2391 ... Recess portion
2392 ... Extending portion
2394 ... Shaft
2395 ... End portion

**Claims**

1. A manipulator attachable to and detachable from a medical device, comprising:

a manipulator main body having a distal end portion attached with an end effector and a proximal end portion connectable to the medical device; and
a mechanical counter mechanism disposed on the manipulator main body, wherein
the counter mechanism
shifts from a first state to a second state by a first operation for the counter mechanism,

shifts from the second state to the first state by a second operation for the counter mechanism, and

counts the shifts from the second state to the first state by the second operation.

2. The manipulator according to claim 1, wherein the counter mechanism comprises:

a counter disk that indicates a count number on its main surface and is rotatably fixed to the manipulator main body;

a first member that is rotatably fixed to the manipulator main body and rotates to one side in a rotational direction to rotate the counter disk, and rotates to the other side not to rotate the counter disk; and

a second member that engages with the first member to restrict the first member to rotate to the other side, wherein

in the first state, the second member and the first member engage with each other and the first member rotates relatively to the one side, and

in the second state, the second member and the first member do not engage with each other and the first member rotates relatively to the other side.

3. The manipulator according to claim 2, wherein

in the first state of the counter mechanism, an end portion of the first member is accommodated in the manipulator main body to allow the proximal end portion of the manipulator to be connected with the medical device, and

in the second state of the counter mechanism, the end portion of the first member protrudes outside the manipulator main body to prevent the proximal end portion of the manipulator from being connected to the medical device.

4. The manipulator according to any one of claims 1 to 3, wherein

when the shifts from the second state to the first state reach a predetermined count, the counter mechanism is prevented from transitioning from the second state to the first state.

5. The manipulator according to any one of claims 1 to 4, wherein

the counter mechanism further has an indicating portion that indicates the count of the shifts.

Fig. 1

EP 4 582 045 A1

# Fig. 2

# Fig. 3

## Fig. 4

NEUTRAL MODE — M1

ROTATE THE DIAL

MOVE THE HANDLE
TO THE END PORTION

MOVE THE HANDLE
TO THE CENTER

PULL THE HANDLE／
PUSH THE BUTTON

WASHING MODE — M2

GEAR DRIVING
MODE — M3

# Fig. 5

# Fig. 6

# Fig. 7

EP 4 582 045 A1

# Fig. 8

## Fig. 9A

## Fig. 9B

## Fig. 9C

## Fig. 9D

## Fig. 10A

## Fig. 10B

# Fig. 11

# Fig. 12A

# Fig. 12B

## Fig. 13A

## Fig. 13B

# Fig. 14

# Fig. 15

## Fig. 16A

## Fig. 16B

## Fig. 16C

## Fig. 17A

## Fig. 17B

# Fig. 18

## Fig. 19A

## Fig. 19B

## Fig. 19C

## Fig. 20

# Fig. 21

## Fig. 22

# Fig. 23

# Fig. 24

## INTERNATIONAL SEARCH REPORT

| International application No. |
|---|
| **PCT/JP2022/032883** |

| A. | CLASSIFICATION OF SUBJECT MATTER |
|---|---|

*A61B 34/37*(2016.01)i
FI:   A61B34/37

According to International Patent Classification (IPC) or to both national classification and IPC

| B. | FIELDS SEARCHED |
|---|---|

Minimum documentation searched (classification system followed by classification symbols)

A61B34/37

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2012-192103 A (OLYMPUS CORP.) 11 October 2012 (2012-10-11) | 1, 4, 5 |
| | paragraphs [0010]-[0055], fig. 1, 4, 6 | |
| A | paragraphs [0010]-[0055], fig. 1, 4, 6 | 2-3 |
| A | JP 2011-194129 A (TERUMO KABUSHIKI KAISHA) 06 October 2011 (2011-10-06) | 1-5 |
| | paragraphs [0023]-[0089] | |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 October 2022** | **01 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/032883**

| Patent document<br>cited in search report | | | Publication date<br>(day/month/year) | Patent family member(s) | | | Publication date<br>(day/month/year) |
|---|---|---|---|---|---|---|---|
| JP | 2012-192103 | A | 11 October 2012 | US | 2013/033186 | A1 | |
| | | | | paragraphs [0055]-[0121] | | | |
| | | | | WO | 2012/124831 | A1 | |
| | | | | CN | 103429187 | A | |
| JP | 2011-194129 | A | 06 October 2011 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020032452 A
- JP 2011194129 A
- JP 5830258 B
- JP 6472532 B